# FASCICULE DE BREVET EUROPEEN

(11) **EP 0 526 603 B1**
(45) Date de publication et mention de la délivrance du brevet: **04.12.1996**
(21) Numéro de dépôt: 92903806.5
(22) Date de dépôt: 19.02.1992
(51) Int. Cl.: C07F 15/00, C07F 15/02, C07F 9/00, G01N 27/30

(54) **Utilisation de complexes mono, bis ou tris (2,2'-bipyridine substituée) d'un métal de transition comme capteurs ampérométriques**
Verwendung von Mono-, Bis- oder Tris-(Substituierte 2,2'-Bipyridin) Übergangsmetallkomplexen als Übermittler für ein amperiometrisches Signaleingabeglied
Use of Mono, Bis or Tris (2,2'-substituted Bipyridine) complexes of a transition metal as mediators for current sensors

(30) Priorité: 21.02.1991 FR 9102199
(43) Date de publication de la demande: 10.02.1993
(73) Titulaire: ASULAB S.A., CH-2501 Bienne (CH)
(72) Inventeur: GRÄTZEL, Michael, CH-1025 St-Sulpice (CH); FRASER, David, CH-1800 Vevey (CH); NAZEERUDDIN, Mohammed, Khaja, CH-2011 Chavannes (CH); ZAKEERUDDIN, Shaik, Mohammed, CH-1020 Renens (CH)
(74) Mandataire: Thérond, Gérard Raymond
(86) Numéro de dépôt international: CH9200033
(87) Numéro de publication internationale: WO9214741

(56) Documents cités:
- EP-A- 0 074 807
- EP-A- 0 178 450
- WO-A-86/02734
- WO-A-90/05732
- GB-A- 956 242
- ORGANIC MAGNETIC RESONANCE, vol. 22, no. 6, juin 1984; M.J. COOK et al., pp. 388-394
- CHEMICAL ABSTRACTS, vol. 53, Columbus, OH (US); G.F. SMITH et al., no. 11087d
- MONATSHEFTE FÜR CHEMIE, vol. 119, no. 1, 1988; W. NUSSBAUMER et al.: pp. 1-15
- CHEMICAL ABSTRACTS, vol. 113, 1990, Columbus, OH (US); L. DELLA CIANA et al., p. 814, no. 90292a
- CHEMICAL ABSTRACTS, vol. 101, 1984, Columbus, OH (US); K.J. TAKEUCHI et al., p. 574, no. 16171w
- JOURNAL OF THE CHEMICAL SOCIETY, Perkin Transactions II, vol. 8, 1984; M.J. COOK et al., pp. 1293-1301
- JOURNAL OF THE CHEMICAL SOCIETY, Perkin Transactions II, vol. 8, 1984; M.J. Cook et al., p 1303-1307
- CHEMICAL ABSTRACTS, vol. 100, 1984, Columbus, OH (US); E.C. CONSTABLE, p. 701, no. 150011b
- JOURNAL OF THE CHEMICAL SOCIETY, Chemical Communications, no. 13, 1988; M.K. NAZEERUDDIN et al., pp. 872-874

## Description

La présente invention concerne une nouvelle application en tant que médiateur pour capteur ampérométrique d'une famille de complexes mono, bis ou tris (2,2' - bipyridine substituée) d'un métal choisi parmi le fer (II), le ruthénium (II), l'osmium (II) ou le vanadium (II), la bipyridine étant substituée par au moins un groupe donneur d'électrons. Cette invention concerne également les procédés de préparation de ces complexes.

L'invention concerne également en tant que composés chimiques nouveaux une famille plus restreinte de complexes de l'osmium comportant trois ligands 2,2' - bipyridine substitués, identiques ou différents.

La détermination de la concentration de certains composants et notamment du glucose dans les liquides biologiques a été considérablement améliorée par l'emploi de capteurs ampérométriques utilisant une électrode revêtue d'un médiateur et d'une enzyme spécifique dudit composant, par exemple la glucose oxydase pour la détection du glucose. Ces capteurs permettent en effet de mesurer le transfert d'électrons intervenant entre l'enzyme et l'électrode, par l'intermédiaire du médiateur, ce transfert d'électrons étant proportionnel à la quantité de composant se trouvant dans l'échantillon de liquide biologique à tester.

La qualité de ces capteurs, c'est-à-dire leur précision, la reproductibilité des résultats donnés par plusieurs capteurs d'une même série, leur fiabilité et la rapidité de leur temps de réponse dépend largement du médiateur utilisé.

On connaissait jusqu'à présent, par exemple d'après le brevet US 4 545 382 de la société Genetics International, des médiateurs tels que le polyviologène, le chloranile, le fluoranile ou le ferrocène.

Toutefois ces médiateurs présentent un certain nombre d'inconvénients.

Ils ne transfèrent pas suffisamment rapidement les électrons entre l'enzyme et l'électrode et de ce fait le temps de réponse du capteur est assez long. De plus, certains de ces médiateurs comme le ferrocène sont assez volatils ou instables particulièrement lorsqu'ils sont exposés à la lumière et les capteurs doivent être conservés sous des conditions assez strictes de lumière et de température. En outre, certains médiateurs notamment le ferrocène, se décomposent dans l'eau par hydrolyse ce qui est gênant lorsque les capteurs sont utilisés dans le sang.

Enfin, l'oxygène entre en compétition avec certains de ces médiateurs et les résultats des mesures de la concentration en glucose varient suivant la qualité d'oxygène présente dans le sang. Ceci peut être gênant suivant que l'on travaille sur du sang veineux ou du sang artériel.

En conséquence, il était souhaitable de chercher, et de préparer des médiateurs rapides, stables et n'ayant pas de problèmes d'interférence avec l'oxygène.

Cette recherche de médiateurs a conduit à sélectionner une famille de complexes mono, bis ou tris (2,2' - bipyridine substituée) d'un métal choisi parmi le fer, le ruthénium, l'osmium ou le vanadium, la bipyridine étant substituée par au moins un groupe donneur d'électrons.

On connait déjà un certain nombre de complexes d'un métal de transition avec au moins un ligand bipyridine substitué ou non, lesdits complexes étant proposés dans des applications très variées, à l'exclusion de toute référence à une application possible comme médiateurs dans un capteur ampérométrique. En raison de leurs propriétés luminescentes, un grand nombre de ces complexes a été proposé comme marqueurs ou comme composants de capteurs solaires. Leur usage comme marqueurs est par exemple évoqué dans les documents ci-après : WO-A-86/02 734; WO-A-90/05 732, EP-A-0 178 450 et ANALYST 83, 661-6 (1958) G.F. SMITH et al. Leur usage comme composants de capteur solaire est par exemple évoqué dans les documents ci-après : EP-A-0 074 807 et Organic Magnetic Resonance, vol. 22, no. 6, juin 1984, M.J. COOK et al.; J. Chem. Soc. Perkin Trans., vol. 8, 1984, pages 1293-1307, M.J. COOK et al. Les variations des propriétés luminescentes et leur corrélation avec les substitutions sur la bipyridine sont par exemple rapportées dans le document, INORG. CHEM. 1990, 29(15), 2792-8 L. DELLA CIANA et al. et INORG. CHEM. 1984, 23(13), 1845-51, K.J. TAKEUCHI et al. D'autres documents concernent leurs propriétés catalytiques dans les réactions chimiques tels que : Monatshefte für Chemie, vol. 119, no. 1, 1988, W. NUSSBAUMER et al.; INORG. CHIM. ACTA 1984, 82(1), 53-7, E.C. CONSTABLE et J. Chem. Soc. Chemi. Commun., no. 13, pages 872-874, 1988, M.K. NAZEERUDDIN. Dans le brevet GB-A-956 242, des complexes de 1, 10 phénanthroline ou de bipyridine substituées sont revendiqués pour leurs propriétés antibactériennes. On observera enfin qu'aucun des documents précités ne décrit de complexes dans lesquels l'osmium comporte trois ligands bipyridines substitués.

L'invention a donc pour objet une nouvelle application en tant que médiateurs pour capteurs ampérométriques, de complexes de 2,2'-bipyridines substitués d'un métal de transition, et à titre des composés chimiques nouveaux des complexes tris (2,2'-bipyridine substituée) d'osmium.

Cette famille de complexes présente un intérêt non seulement du point de vue théorique, mais surtout en raison de ses bonnes propriétés de médiateur et des nombreuses autres applications pratiques qu'elle pourrait avoir.

L'invention sera mieux comprise à la lecture de la description suivante de la famille de complexes et de plusieurs modes de réalisation des procédés de préparation de ces complexes.

La famille de complexes mono, bis ou tris (2,2' - bipyridine substituée) d'un métal M, présente la formule générale (i) suivante : 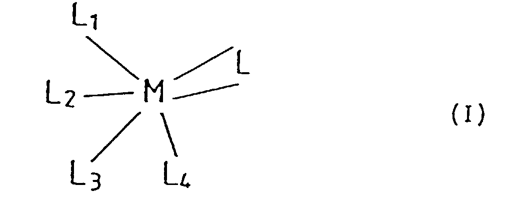 dans laquelle M est choisi parmi le fer, l'osmium, le ruthénium ou le vanadium.

L représente une bipyridine substituée par au moins un groupe donneur d'électrons, conférant au complexe les propriétés d'oxydoréduction souhaitées pour un médiateur. Ce groupe donneur d'électrons est de préférence choisi parmi un groupe OH, un groupe alkoxy, un groupe aryloxy ou un groupe amine primaire, secondaire ou tertiaire.

L₁, L₂, L₃, L₄ sont des ligands formant un complexe de coordination avec le fer, l'osmium, le ruthénium ou le vanadium.

On notera également que dans ces complexes, le ligand L représente de préférence, une bipyridine bisubstituée en position 4,4' et plus particulièrement la 4,4'- dihydroxy - 2,2' - bipyridine, la 4,4' - diméthoxy - 2,2' - bipyridine, ou la 4,4' - bis (N,N - éthylamino) - 2,2' - bipyridine connue également sous le nom de 4,4' - bis (N,N - diéthylamino) - 2,2' - bipyridine, les complexes présentant alors les formules générales suivantes, référencées (II), (III) et (IV) respectivement :

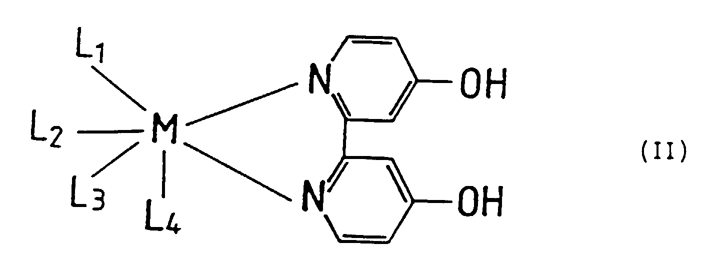

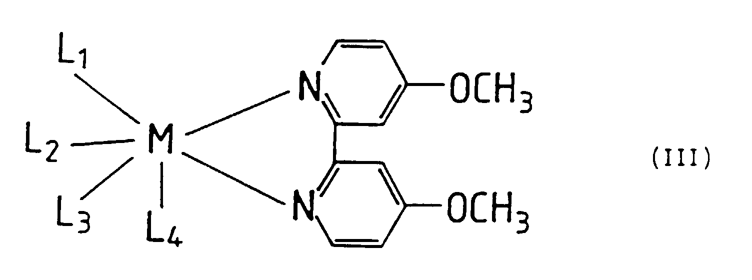

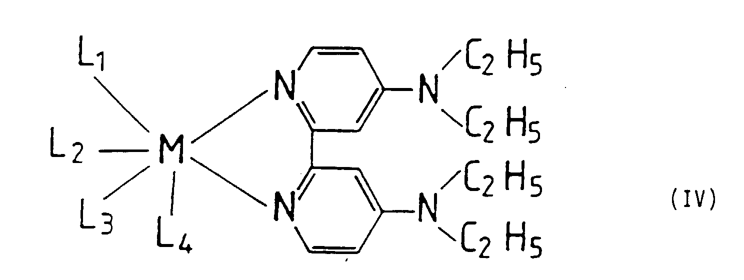

Les ligands L₁, L₂, L₃ et L₄ sont des ligands mono, bi, tri ou tétradentés et peuvent se combiner de la façon suivante :
- soit L₁, L₂, L₃ et L₄ représentent chacun un ligand monodenté et sont totalement ou partiellement identiques entre eux ou différents les uns des autres,
- soit L₁ et L₂ forment ensemble un ligand bidenté et L₃ et L₄ sont identiques ou différents et représentent chacun un ligand monodenté,
- soit L₁ et L₂ d'une part et L₃ et L₄ d'autre part forment respectivement un ligand bidenté,
- soit L₁ représente un ligand monodenté et L₂, L₃ et L₄ forment ensemble un ligand tridenté,
- soit L₁, L₂, L₃ à L₄ forment ensemble un ligand tétradenté. Parmi les ligands monodentés que l'on peut utiliser, on citera : CN⁻, Cl⁻, Br⁻, I⁻, SCN⁻, H₂O, NH₃, la triphénylphosphine, les trialkylphosphines, les amines primaires, secondaires ou tertaires, la pyridine ou les pyridines substituées par Cl, NH₂, NO₂ ou par un groupe alkyle.

Parmi les ligands bidentés que l'on peut utiliser, on citera : l'éthylène diamine, le 1,2 - bis (2 - pyridyl) éthane, l'acide oxalique O⁻-CO-CO-O⁻, l'acétylacétone CH₃-CO-CH₂-CO-CH₃, la glycine NH₂-CH₂-COO⁻, et de préférence, les bipyridines et les phénanthrolines bisubstituées par un groupe R₁ et un groupe R₂, R₁ et R₂ pouvant être identiques ou différents et représentant chacun l'hydrogène, NO₂, Cl, un groupe alkyle, un groupe aryle, un groupe OH, un groupe alkoxy, un groupe aryloxy ou un groupe amine primaire, secondaire ou tertiaire.

Parmi les ligands tridentés, on utilisera de préférence la terpyridine de formule générale (V) 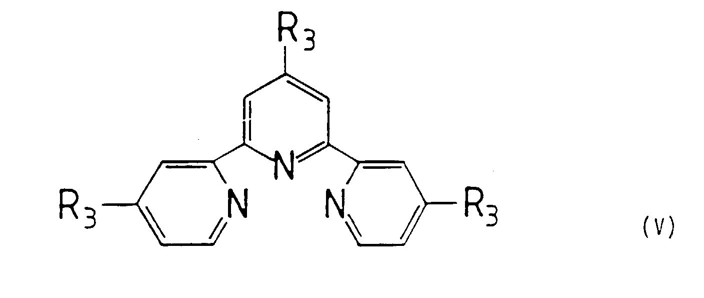 dans laquelle R₃ représente l'hydrogène ou un groupe alkyle.

Parmi les ligands tétradentés, on utilisera de préférence - le triéthylène tétraamine : 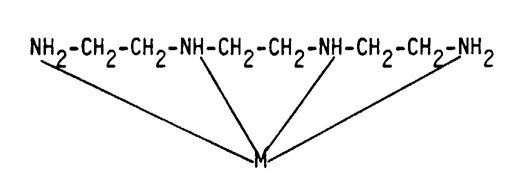 schématisé ci-dessus en coordination avec le métal M, et - l'acide éthylène diamine diacétique: 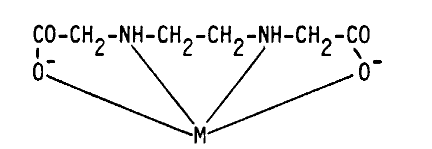 schématisé également en coordination avec le métal M.

Lorsque L₁ et L₂ d'une part et L₃ et L₄ d'autre part forment respectivement un ligand bidenté et que ces deux ligands bidentés sont identiques au ligand L, on obtient un complexe tris (ligand L) d'un métal M choisi parmi le fer, le ruthénium, l'osmium ou le vanadium.

Les complexes tris (ligand L) préférés de la présente invention sont :
- les complexes tris (4,4' - dihydroxy - 2,2'- bipyridine) du métal M, et
- les complexes tris (4,4' - diméthoxy - 2,2' - bipyridine) du métal M, et
- les complexes tris (4,4' - bis (N,N - éthylamino) - 2,2'-bipyridine) du métal M.

Lorsque L₁ et L₂ forment ensemble un premier ligand bidenté et que L₃ et L₄ forment ensemble un second ligand bidenté et que de plus seul le premier ligand bidenté est identique au ligand L, on obtient un complexe bis (ligand L) du métal M, M étant tel que défini ci-dessus.

Les complexes bis (ligand L) préférés de la présente invention sont :
- les complexes bis (4,4' - dihydroxy - 2,2' - bipyridine) mono (2,2' - bipyridine) du métal M,
- les complexes bis (4,4' - dihydroxy - 2,2' - bipyridine) mono (4,4' - diméthoxy - 2,2' - bipyridine) du métal M,
- les complexes bis (4,4' - dihydroxy - 2,2' - bipyridine) mono (4,4' - diméthyl - 2,2' - bipyridine) du métal M,
- les complexes bis (4,4' - dihydroxy - 2,2' - bipyridine) mono (4 - alkyl - 2,2' - bipyridine) du métal M,
- les complexes bis (4,4' - dihydroxy - 2,2' - bipyridine) mono (4,4' - dialkyl - 2,2' - bipyridine) du métal M,
- les complexes bis (4,4' - dihydroxy - 2,2' - bipyridine) mono (4,7 - dihydroxy - 1,10 phénanthroline) du métal M,
- les complexes bis (4,4' - dihydroxy - 2,2' - bipyridine) mono (4,4' - bis (N,N - éthylamino) - 2,2' - bipyridine) du métal M,
- les complexes bis (4,4' - diméthoxy - 2,2' - bipyridine) mono (2,2' - bipyridine) d'un métal M,
- les complexes bis (4,4' - diméthoxy - 2,2' - bipyridine) mono (4,4' - diméthyl - 2,2' - bipyridine) du métal M,
- les complexes bis (4,4' - diméthoxy - 2,2' - bipyridine) mono (4 - alkyl - 2,2' - bipyridine) du métal M,
- les complexes bis (4,4' - diméthoxy - 2,2' - bipyridine) mono (4,4' - dialkyl - 2,2' - bipyridine) du métal M,
- les complexes bis (4,4' - diméthoxy - 2,2' - bipyridine) mono (4,7 - dihydroxy - 1, 10 - phénanthroline) du métal M,
- les complexes bis (4,4' - diméthoxy - 2,2' - bipyridine) mono (4,4' - dihydroxy - 2,2' - bipyridine) du métal M,
- les complexes bis (4,4' - diméthoxy - 2,2' - bipyridine) mono (4,4' - bis (N,N - éthylamino) - 2,2' - bipyridine) du métal M,
- les complexes bis (4,4' - bis (N,N - éthylamino) - 2,2' - bipyridine) mono (2,2' - bipyridine) du métal M,
- les complexes bis (4,4' - bis (N,N - éthylamino) - 2,2' - bipyridine) mono (4,4' - diméthoxy - 2,2' - bipyridine) du métal M,
- les complexes bis (4,4' - bis (N,N' - éthylamino) - 2,2' - bipyridine) mono (4,4' - diméthyl - 2,2' - bipyridine) du métal M,
- les complexes bis (4,4' - bis (N,N - éthylamino) - 2,2' - bipyridine) mono (4 - alkyl - 2,2' - bipyridine) du métal M,
- les complexes bis (4,4' - bis (N,N - éthylamino) - 2,2' - bipyridine) mono (4,4' - dialkyl - 2,2' - bipyridine) du métal M,
- les complexes bis (4,4' - bis (N,N - éthylamino) - 2,2' - bipyridine) mono (4,4' - dihydroxy - 2,2' - bipyridine) du métal M,
- les complexes bis (4,4' - bis (N,N - éthylamino) - 2,2' - bipyridine) mono (4,7 - dihydroxy - 1, 10 - phénanthroline) du métal M.

Lorsque L₁ et L₂ forment ensemble un premier ligand bidenté et que L₃ et L₄ forment ensemble un second ligand bidenté et que de plus ce premier et ce second ligand sont différents du ligand L, on obtient un complexe mono (ligand L) d'un métal M choisi parmi le fer, le ruthénium, l'osmium ou le vanadium.

Lorsque ce premier ligant (L₁, L₂) et ce second ligand (L₃, L₄) sont identiques, les complexes mono (ligand L) préférés de la présente invention, sont les suivants :
- les complexes mono (4,4' - dihydroxy - 2,2' - bipyridine) bis (4,4' - diméthyl - 2,2' - bipyridine) du métal M,
- les complexes mono (4,4' - dihydroxy - 2,2' - bipyridine) bis (2,2' - bipyridine) du métal M,
- les complexes mono (4,4' - dihydroxy - 2,2' - bipyridine) bis (4 - alkyl - 2,2' - bipyridine) du métal M,
- les complexes mono (4,4' - dihydroxy - 2,2' - bipyridine), bis (4,7 - dihydroxy - 1,10 - phénanthroline) du métal M,
- les complexes mono (4,4' - diméthoxy - 2,2' - bipyridine) bis (4,4' - diméthyl - 2,2' - bipyridine) du métal M,
- les complexes mono (4,4' - diméthoxy - 2,2' - bipyridine) bis (2,2' - bipyridine) du métal M,
- les complexes mono (4,4' - diméthoxy - 2,2' - bipyridine) bis (4 - alkyl - 2,2' - bipyridine) du métal M,
- les complexes mono (4,4' - diméthoxy - 2,2' - bipyridine) bis (4,7 - dihydroxy - 1, 10 - phénanthroline) du métal M,
- les complexes mono (4,4' - bis (N,N - éthylamino) - 2,2' - bipyridine) bis (4,4' - diméthyl - 2,2' - bipyridine) du métal M,
- les complexes mono (4,4' - bis (N,N - éthylamino) - 2,2' - bipyridine) bis (2,2' - bipyridine) du métal M,
- les complexes mono (4,4' - bis (N,N - éthylamino) - 2,2' - bipyridine) bis (4 - alkyl - 2,2' - bipyridine) du métal M,
- les complexes mono (4,4' - bis (N,N - éthylamino) - 2,2' - bipyridine) bis (4,7 - dihydroxy - 1,10 - phénanthroline) du métal M,

Lorsque ledit premier ligand (L₁, L₂) et ledit second ligand (L₃, L₄) sont différents, les complexes mono (ligand L) préférés sont les suivants :
- les complexes mono (4,4' - diméthoxy - 2,2' - bipyridine) mono (4,4' - dihydroxy - 2,2' - bipyridine) mono (4,4' - diméthyl - 2,2' bipyridine) du métal M.

La présente invention fournit aussi des procédés de préparation des complexes décrits précédemment.

L'invention concerne notamment un procédé de préparation d'un complexe tris (ligand L) d'un métal M choisi parmi le fer, le ruthénium, l'osmium ou le vanadium. Le ligand L est un ligand bidenté choisi parmi la 4,4' - diméthoxy - 2,2' - bipyridine, la 4,4' - dihydroxy - 2,2' - bipyridine ou la 4,4' - bis (N,N - éthylamino) - 2,2' - bipyridine.

De manière générale, ce procédé consiste à :
- faire réagir un sel soluble du métal M avec une quantité sensiblement stoechiométrique d'un ligand A en présence de solvants appropriés pour dissoudre ce ligand, puis à,
- faire refluer cette solution sous une atmosphère d'azote, à la pression atmosphérique et à une température voisine de la température d'ébullition de ces solvants.

Le ligand A est dans la plupart des cas identique au ligand L ou en est suffisamment proche pour se transformer en ce ligand L, au cours de la réaction. Le ligand A peut, par exemple, être de même nature que le ligand L, mais être substitué différemment. Ceci est le cas dans l'exemple 1, ou l'on part d'une bipyridine bisubstituée par deux groupes méthoxy pour aboutir à une bipyridine bisubstituée par deux groupes hydroxy. Dans l'exemple 1, ainsi que dans les exemples 5 et 11 décrits ci-après, on utilise la 4,4' - diméthoxy - 2,2' - bipyridine pour otenir des complexes avec la 4,4' - dihydroxy - 2,2' - bipyridine, car cette dernière n'est pas disponible commercialement, compte tenu des difficultés et des coûts de fabrication.

La nature du sel soluble du métal M varie en fonction de ce dernier. Suivant que le métal M est du fer, du ruthénium, de l'osmium ou du vanadium, les sels solubles sont respectivement du FeCl₂, du K₂OsCl₆, du RuCl₃ ou du VCl₃.

L'invention concerne premièrement un procédé de préparation d'un complexe tris (4,4' - dihydroxy - 2,2' - bipyridine) d'un métal M choisi parmi le fer, le ruthénium, l'osmium ou le vanadium.

De manière générale, ce procédé consiste à :
- faire réagir un sel soluble du métal M, M étant tel que défini ci-dessus, avec une quantité sensiblement stoechiométrique de 4,4' - diméthoxy - 2,2' - bipyridine, en présence d'éthylène glycol, puis à,
- faire refluer cette solution sous une atmosphère d'azote, à la pression atmosphérique et à une température comprise entre 190 et 200°C, de préférence à 195°C.

Les sels solubles du métal M sont identiques à ceux qui ont été décrits précédemment.

Le choix du solvant utilisé (éthylène glycol) permet de travailler à des températures élevées (190 à 200°C environ) et de rompre uniquement la liaison entre OCH₃ et la bipyridine, sans que d'autres modifications chimiques n'interviennent.

Une fois que la liaison OCH₃ est rompue, les groupes OH peuvent ensuite se former pour aboutir à un complexe tris (4,4' - dihydroxy - 2,2' - bipyridine) du métal M.

Un exemple précis d'obtention de l'un de ces complexes est donné ci-après.

### Exemple 1

### Préparation du complexe tris (4,4' - dihydroxy - 2,2' - bipyridine) d'osmium.

On dissout 0,100 g (0,21 mmol) de K₂OsCl₆ dans 10 ml d'éthylène glycol et on y ajoute 0,144 g (0,67 mmol) de 4,4' - diméthoxy - 2,2' - bipyridine. Puis on laisse cette solution refluer pendant au moins 24 heures sous une atmosphère d'azote, à la pression atmosphérique et à une température comprise entre 190 et 200°C environ, de préférence à 195°C. Une couleur brune apparaît. Après avoir laissé refroidir la solution à température ambiante, on la concentre à la moitié de son volume d'origine. On précipite le produit sous forme de trifluorométhansulfonate par addition d'acide trifluorométhansulfonique dans la solution d'éthylène glycol.

On filtre le complexe obtenu, on le lave avec un mélange (1:10 vol/vol) d'acétone et diéthyléther puis on le sèche sous vide élevé. On obtient un complexe tris (4,4' - dihydroxy - 2,2' - bipyridine) d'osmium, présentant les propriétés physico-chimiques suivantes :

### - Analyse élémentaire

| Formule : C₃₂H₂₄N₆O₁₂F₆OsS₂ (1,55 H₂O) (mesures faites avec le trifluorométhansulfonate) | | |
|---|---|---|
| | calculé | trouvé |
| C | 35,56 % | 35,75 % |
| H | 2,53 % | 3,02 % |
| N | 7,78 % | 7,10 % |
| H₂O | 2,58 % | 2,58 % |

### - Potentiel d'oxydo-réduction E° (potentiel normal)

Lorsque l'on dissout le complexe dans un solvant organique tel l'acétonitrile en présence de LiCl₄ 0,2 M , et que l'on effectue une mesure potentiodynamique avec une électrode en carbone vitreux, on obtient E° = + 280 mV (par rapport à une électrode de référence au calomel SCE),

Dans un solvant aqueux : PBS (solution de tampon phosphate : NaCl 100mM, NaH₂PO₄ 10mN, ajusté d'abord à pH 7,4, puis à pH1 et à pH13 ) avec une électrode en carbone, on obtient :
- pH = 1 E° = + 150 mV (SCE)
- pH = 13 E° = - 1V (SCE)

### - RMN

L'échantillon dissous dans CD₂Cl₂ ne présente aucun pic OCH₃ à 4,1 ppm, ce qui indique l'hydrolyse complète de ces groupes pour former des groupes OH. On observe des pics de protons aromatiques entre 6,8 ppm et 8 ppm, par rapport à un standard de tétraméthylsilane.

### - Données du spectre d'absorption des ondes UV et visibles

| longueur d'onde λ max (nm) | coefficient d'extinction ε(M⁻¹ cm⁻¹) |
|---|---|
| 239 | 13850 |
| 269 | 13300 |
| 300 | 12600 |
| 357 | 11500 |
| 385 | 10858 |
| 474 | 7430 |
| 512 | 6240 |
| 654 | 1475 |
| 712 | 1156 |

Lorsque le ligand L est la 4,4' - diméthoxy - 2,2' - biypridine, le procédé de préparation du complexe tris (ligand L) de métal M consistera à :
- faire réagir un sel soluble du métal M avec une quantité sensiblement stoechiométrique de 4,4' - diméthoxy - 2,2' - bipyridine en présence de méthanol, de DMF (diméthylformamide) et/ou d'eau, puis à,
- faire refluer cette solution sous une atmosphère d'azote, à la pression atmosphérique et à une température comprise entre 70 et 80°C environ.

Le choix des solvants utilisés (méthanol, DMF et/ou eau) est important car il permet contrairement à l'exemple 1 de travailler à des températures relativement basses (environ 70 à 80°C) et d'éviter ainsi la rupture de la liaison entre le groupe OCH₃ et la bipyridine. De plus, ces solvants permettent de réduire le métal M d'un état d'oxydation + 3 à un état + 2 pour le ruthénium, d'un état + 4 à un état + 2 pour l'osmium et d'un état + 3 à un état + 2 pour le vanadium. Le fer ne change pas d'état d'oxydation, il est déjà dans l'état d'oxydation + 2. Toutefois, on notera que pour le vanadium, il est nécessaire d'utiliser des réactifs supplémentaires pour atteindre l'état d'oxydation 0 désiré.

Un exemple précis d'obtention de l'un de ces complexes est donné ci-après.

### Exemple 2

### Préparation d'un complexe tris (4,4' - diméthoxy - 2,2' - bipyridine) d'osmium.

On dissout 0,100 g (0,21 mmol) de K₂OsCl₆ dans un mélange de 5 ml d'eau, 5 ml de méthanol et 5 ml de DMF. On ajoute à cette solution 0,144 g (0,67 mmol) de 4,4' - diméthoxy - 2,2' - bipyridine et l'on fait refluer l'ensemble pendant au moins 60 heures sous une atmosphère d'azote, à la pression atmosphérique et à une température comprise entre 70 et 80°C environ.

Une couleur brune apparaît. Après refroidissement à température ambiante, on filtre la solution et on élimine complètement le solvant par évaporation rotative. On ajoute alors une quantité minimum d'éthanol pour dissoudre le produit et l'on filtre ensuite la solution pour éliminer le KCl insoluble. On fait ensuite précipiter le produit sous forme de trifluorométhansulfonate par addition d'acide trifluorométhansulfonique dilué. On filtre alors le complexe, puis on le lave avec un mélange 1:10 vol/vol d'acétone et de diéthyléther, et enfin on le sèche sous vide élevé.

On obtient un complexe tris (4,4' - diméthoxy - 2,2' - bipyridine) d'osmium présentant les propriétés physico-chimiques suivantes :

### - Analyse élémentaire

| Formule : C₃₈H₃₆N₆O₁₂F₆OsS₂ (1,11 H₂O) (mesure faite avec le trifluorométhansulfonate) | | |
|---|---|---|
| | calculé | trouvé |
| C | 39,45 % | 39,13 % |
| H | 3,33 % | 3,27 % |
| N | 7,26 % | 7,41 % |
| H₂O | 1,73 % | 1,73 % |

### - Potentiel d'oxydo-réduction E° (potentiel normal)

Lorsque l'on dissout le complexe dans un solvant organique tel l'acétonitrile, en présence de LiClO₄ 0,2 M , et que l'on effectue une mesure potentiodynamique avec une électrode en carbone vitreux, on obtient E° = + 325 mV, par rapport à une électrode de référence au calomel (SCE).
Dans un solvant aqueux PBS (solution de tampon phosphate : NaCl 100mM, NaH₂PO₄ 10 mM, ajusté à pH 7,4), avec une électrode en carbone, on obtient E° = + 225 mV.

### - RMN

L'échantillon dissous dans CD₂Cl₂ présente un pic OCH₃ à 4,1 ppm et des pics de protons aromatiques entre 6,8 ppm et 8,0 ppm, par rapport à un standard de tétraméthylsilane.

### - Données du spectre d'absorption des ondes UV et visibles

| longueur d'onde λ max (nm) | coefficient d'extinction ε(M⁻¹ cm⁻¹) |
|---|---|
| 228 | 80800 |
| 271 | 70800 |
| 333 | 22236 |
| 385 | 16012 |
| 397 | 15296 |
| 470 | 12564 |
| 510 | 11404 |
| 630 | 4820 |
| 696 | 4012 |

Le procédé de préparation général d'un complexe tris (4,4' - bis (N,N - éthylamino) - 2,2' - bipyridine) d'un métal M, choisi parmi le fer, le ruthénium, l'osmium ou le vanadium va maintenant être décrit.

Ce procédé consiste à :
- faire réagir un sel soluble du métal M avec une quantité sensiblement stoechiométrique de 4,4' - bis (N,N - éthylamino) - 2,2' - bipyridine, en présence d'éthylène glycol, puis à,
- faire refluer cette solution sous une atmosphère d'azote, à la pression atmosphérique et à une température comprise entre 190 et 200°C environ, de préférence à 195°C.

Le sel soluble de métal M est identique à ceux décrits précédemment.

Un exemple précis d'obtention de l'un de ces complexes est donné ci-après.

### Exemple 3

### Préparation d'un complexe tris (4,4' - bis (N,N - éthylamino) - 2,2' - bipyridine) d'osmium.

On dissout 0,100 g (0,21 mmol) de K₂OsCl₆ dans 10 ml d'éthylène glycol et on y ajoute 0,193 g (0,64 mmol) de 4,4' - bis (N,N - éthylamino) - 2,2' - bipyridine. On laisse refluer cette solution pendant au moins 10 heures, sous une atmosphère d'azote, à la pression atmosphérique et à une température comprise entre 190 et 200°C environ, de préférence à 195°C. Après avoir laissé refroidir la solution à température ambiante, on la filtre et on concentre le solvant à la moitié de son volume d'origine. On précipite le produit sous forme de trifluorométhansulfonate par addition d'acide trifluorométhansulfonique.

On filtre le complexe obtenu, on le lave avec un mélange (1:10 vol/vol) d'acétone et de diéthyléther et on le sèche sous vide élevé.

La présente invention concerne également la préparation de complexe bis (ligand L) mono (ligand B) d'un métal M choisi parmi le fer, l'osmium, le ruthénium ou le vanadium, le ligand L étant choisi parmi la 4,4' - diméthoxy - 2,2' - bipyridine, la 4,4' - dihydroxy - 2,2' - bipyridine ou la 4,4' - bis (N,N - éthylamino) - 2,2' - bipyridine) et le ligand B étant un ligand bidenté tels que ceux cités au début de la description.

La préparation de ce complexe nécessite l'emploi d'un complexe bis (ligand L) Cl₂ dudit métal M.

Un mode de réalisation de ce complexe (bis ligand L) Cl₂ dudit métal M va maintenant être décrit. Toutefois, il est bien évident que le complexe bis (ligand L) Cl₂ du métal M utilisé dans la préparation du complexe bis (ligand L) mono (ligand B) du métal M, ne nécessite pas obligatoirement d'être préparé comme décrit ici.

La préparation du complexe bis (ligand L) Cl₂ d'un métal M choisi par le fer, l'osmium, le ruthénium ou le vanadium, le ligand L étant choisi parmi la 4,4' - diméthoxy - 2,2' - bipyridine ou la 4,4' - dihydroxy - 2,2' - bipyridine, consiste de manière générale à :
- faire réagir un sel soluble du métal M, (M étant tel que défini ci-dessus), avec une quantité sensiblement stoechiométrique de 4,4' - diméthoxy - 2,2' - bipyridine en présence d'un mélange de solvants appropriés, notamment par exemple, l'eau et le méthanol dans le cas du premier ligand L et l'éthylène glycol dans le deuxième cas, puis à,
- faire refluer cette solution sous une atmosphère d'azote, à la pression atmosphérique et par exemple, à une température comprise entre 70 et 80°C si l'on utilise l'eau et le méthanol ou à une température comprise entre 190 et 200°C si l'on utilise l'éthylène glycol.

La préparation de ce même complexe lorsque le ligand L est la 4,4' - bis (N,N - éthylamino) - 2,2' - bipyridine consiste de manière générale à :
- faire réagir un sel soluble du métal M, M étant tel que défini ci-dessus, avec une quantité sensiblement stoechiométrique de 4,4' - bis (N, N - éthylamino) - 2,2' - bipyridine en présence de DMF, puis à
- faire refluer cette solution sous une atmosphère d'azote, à la pression atmosphérique et à une température comprise entre 150 et 155°C.

Dans ces deux cas, les sels solubles de métal M sont tels que décrits précédemment.

Trois exemples de préparation plus précis sont donnés ci-après.

### Exemple 4

### Préparation d'un complexe bis (4,4' - diméthoxy - 2,2' - bipyridine) Cl₂ d'osmium.

On dissout 0,100 g (0,21 mmol) de K₂OsCl₆ dans un mélange de 5 ml d'eau et 5 ml de méthanol et on ajoute à la solution 0,90 g (0,42 mmol) de 4,4' - diméthoxy - 2,2' - bipyridine. On laisse refluer la solution pendant au moins 2 heures, sous une atmosphère d'azote, à la pression atmosphérique et à une température comprise entre 70 et 80°C. Une couleur violet foncé apparaît. On laisse refroidir la solution à température ambiante et on élimine complètement le solvant par évaporation rotative. On ajoute alors 10 ml d'eau. Ensuite, on isole le complexe bis (4,4' - diméthoxy - 2,2' - bipyridine) Cl₂ d'osmium par filtration, on le lave avec de l'eau puis avec un mélange d'acétone et de diéthyléther (1:6 vol/vol) pour éliminer les traces de ligand qui n'ont pas réagi. Enfin, on sèche ce complexe sous vide élevé.

Les résultats de l'analyse élémentaire de ce produit sont donnés ci-après.

### - Analyse élémentaire

| Formule : C₂₄H₂₄N₄O₄OsCl₂ | | |
|---|---|---|
| | calculé | trouvé |
| C | 41,56 % | 42,02 % |
| H | 3,49 % | 3,73 % |
| N | 8,08 % | 8,45 % |

### Exemple 5

### Préparation d'un complexe bis (4,4' - dihydroxy - 2,2' - bipyridine) Cl₂ d'osmium.

On dissout 0,100 g (0,21 mmol) de K₂OsCl₆ dans un mélange de 2 ml d'eau et de 10 ml d'éthylène glycol et on ajoute à la solution 0,090 g (0,42 mmol) de 4,4' - diméthoxy - 2,2' - bipyridine. On laisse refluer la solution pendant au moins 16 heures sous une atmosphère d'azote, à la pression atmosphérique et à une température comprise entre 190 et 200°C, de préférence 195°C. Une couleur violet foncé apparaît. On refroidit la solution à température ambiante puis on la concentre par évaporation rotative. On précipite le produit obtenu par addition d'acétone. On isole le précipité par filtration, on le lave avec de l'acétone et de l'éther puis on le sèche sous vide élevé.

Le complexe bis (4,4' - dihydroxy - 2,2' - bipyridine) Cl₂ d'osmium ainsi obtenu se présente sous forme de cristaux bleus. Le rendement est de 80 à 90 %.

Dans ces deux exemples 4 et 5 le choix des solvants et des températures de réaction a été dicté par les mêmes raisons que celles évoquées précédemment.

### Exemple 6

### Préparation d'un complexe bis (4,4' - bis (N,N - éthylamino) - 2,2' - bipyridine) Cl₂ d'osmium.

On dissout 0,100 g (0,21 mmol) de K₂OsCl₆ dans 10ml de DMF et on ajoute à la solution 0,125 g (0,42 mmol) de 4,4' - bis (N,N - éthylamino) - 2,2' - bipyridine.

On laisse refluer la solution pendant au moins 4 heures sous une atmosphère d'azote, à la pression atmosphérique et à une température comprise entre 150 et 155°C. On laisse refroidir la solution à température ambiante et on élimine complètement le solvant par évaporation rotative. On y ajoute 10ml d'eau froide et on isole le complexe bis (4,4' - bis (N,N - éthylamino) - 2,2' - bipyridine) Cl₂ d'osmium (qui est difficilement soluble) par filtration. Puis on lave ce complexe avec de l'eau froide puis avec un mélange (1:6 vol/vol) d'acétone et de diéthyléther pour éliminer les traces de ligand qui n'ont pas réagi, puis on le sèche sous vide élevé.

On va maintenant décrire la préparation du complexe bis (ligand L) mono (ligand B) d'un métal M, évoqué précédemment.

Ce procédé consiste à :
- faire réagir un complexe bis (ligand L) Cl₂ dudit métal M, avec une quantité sensiblement stoechiométrique de ligand B, en présence de solvants appropriés pour dissoudre ce complexe et le ligand B, puis à,
- faire refluer la solution sous une atmosphère d'azote, à la pression atmosphérique et à une température voisine de la température d'ébullition de ces solvants.

Toutefois, on notera comme précédemment qui si le ligand B est la 4,4' - dihydroxy - 2,2' - bipyridine on fera de préférence réagir le complexe chloré avec la 4,4' - diméthoxy - 2,2' - bipyridine, puisque celle-ci est plus facilement disponible sur le marché.

Trois exemples de réalisation plus précis vont maintenant être décrits.

### Exemple 7

### Préparation d'un complexe bis (4,4' - diméthoxy - 2,2' - bipyridine) mono (4,4' - diméthyl - 2,2' - bipyridine) d'osmium.

On fait réagir 0,05 g (0,07 mmol) d'un complexe bis (4,4' - diméthoxy - 2,2' - bipyridine) Cl₂ d'osmium avec 0,017 g (0,09 mmol) de 4,4' - diméthyl - 2,2' - bipyridine en présence d'un mélange de 10 ml de DMF (diméthylformamide), 10 ml d'eau et 10 ml de méthanol. On fait refluer pendant au moins 40 heures sous une atmosphère d'azote à la pression atmosphérique et à une température comprise entre 70 et 80°C. Ensuite, on laisse refroidir le mélange réactionnel à température ambiante, on le filtre et on élimine complètement le solvant par évaporation rotative. On dissout alors le produit dans un minimum d'éthanol et on le précipite sous forme de trifluorométhansulfonate par addition d'acide trifluorométhansulfonique. Enfin, on filtre le précipité, on le lave avec un mélange d'acétone et de diéthyléther (1:6 vol/vol) puis on le sèche sous vide élevé.

Ce complexe présente les propriétés physico-chimiques suivantes :

### - Analyse élémentaire

| Formule C₃₈H₃₆N₆O₁₀F₆OsS₂ (0,90H₂O) (mesures faites avec le trifluorométhansulfonate) | | |
|---|---|---|
| | calculé | trouvé |
| C | 40,71 % | 41,03 % |
| H | 3,40 % | 3,46 % |
| N | 7,5 % | 7,58 % |
| F | 10,17 % | 10,24 % |
| H₂O | 1,45 % | 1,44 % |

### - Potentiel d'oxydo-réduction E°(potentiel normal)

Lorsque l'on dissout le complexe dans un solvant organique tel que l'acétonitrile en présence de LiClO₄ 0,2 M et que l'on effectue une mesure potentiodynamique avec une électrode en carbone vitreux : on obtient E° = + 450 mV (référence : électrode au calomel SCE). Dans un tampon phosphate PBS (NaCl 100mM, NaH₂ PO₄ 10 mM, ajusté à pH 7,4), avec une électrode en carbone vitreux, on obtient E° = + 340 mV (référence : électrode au calomel SCE).

### - RMN

L'échantillon dissous dans du CD₂Cl₂ donne un pic pour OCH₃ à 4,1 ppm et un pic pour CH₃ à 2,65 ppm, ainsi que des pics de protons aromatiques entre 6,8 ppm et 8,0 ppm, par rapport à un standard de tétraméthylsilane.

### - Données du spectre d'absorption des ondes UV et visibles

| longueur d'onde λ max (nm) | coefficient d'extinction ε(M⁻¹ cm⁻¹) |
|---|---|
| 216 | 108000 |
| 290 | 62000 |
| 336 | 15520 |
| 378 | 14580 |
| 462 | 13528 |
| 494 | 13376 |
| 628 | 6920 |
| 674 | 6540 |

### Exemple 8

### Préparation d'un complexe bis (4,4' - dihydroxy - 2,2' - bipyridine) mono (4,4' - diméthyl - 2,2' - bipyridine) d'osmium.

On fait réagir 0,05 g (0,07 mmol) de complexe bis (4,4' - dihydroxy - 2,2' - bipyridine) Cl₂ d'osmium avec 0,017 g (0,09 mmol) de 4,4' - diméthyl - 2,2' - bipyridine dans 10 ml d'éthylène glycol, puis on fait refluer l'ensemble pendant au moins 4 heures sous une atmosphère d'azote à la pression atmosphérique et à une température comprise entre 190 et 200°C, de préférence à 195°C. Une couleur brune apparaît. Le mélange réactionnel est refroidi à température ambiante puis le solvant est concentré à la moitié de son volume d'origine. On fait précipiter le produit obtenu sous forme de trifluorométhansulfonate par addition d'acide trifluorométhansulfonique dilué à la solution d'éthylène glycol. Le complexe bis (4,4' - dihydroxy - 2,2' - bipyridine) mono (4,4' - diméthyl - 2,2' - bipyridine) d'osmium obtenu est filtré, lavé avec un mélange d'acétone et de diéthyléther (1:10 vol/vol) et séché sous vide élevé.

### Exemple 9

### Préparation d'un complexe bis (4,4' - bis (N,N - éthylamino) - 2,2' - bipyridine) mono (4,4' - diméthyl - 2,2' - bipyridine) d'osmium.

On fait réagir 0,05 g (0,058 mmol) de complexe bis (4,4' - bis (N, N - éthylamino) - 2,2' - bipyridine) Cl₂ d'osmium avec 0,014 g (0,07 mmol) de 4,4' - diméthyl - 2,2' - bipyridine dans 10 ml d'éthylène glycol, puis on fait refluer cette solution pendant au moins 6 heures sous une atmosphère d'azote, à la pression atmosphérique et à une température comprise entre 190 et 200°C, de préférence à 195°C. On refroidit le mélange réactionnel à température ambiante, puis on le filtre et on concentre le solvant à la moitié de son volume d'origine. On fait précipiter le produit obtenu sous forme de trifluorométhansulfonate par addition d'acide trifluorométhansulfonique. Le précipité est filtré, lavé avec un mélange (1:6 vol/vol) d'acétone et de diéthyléther puis séché sous vide élevé.

Les procédés de préparation qui viennent d'être décrits dans les exemples 7, 8 et 9 pour l'osmium peuvent être généralisés au fer, au ruthénium et au vanadium.

Enfin, l'invention concerne un procédé de préparation d'un complexe mono (ligand L) bis (ligand B) d'un métal M choisi parmi le fer, l'osmium, le ruthénium ou le vanadium. Le ligand L est choisi comme précédemment parmi la 4,4' - diméthoxy - 2,2' - bipyridine, la 4,4' - dihydroxy - 2,2' - bipyridine ou la 4,4' - bis (N,N - éthylamino) - 2,2' - bipyridine et le ligand B est un des ligands bidentés cités au début de la description.

D'une manière générale, ce procédé consiste à :
- faire réagir un complexe bis (ligand B) Cl₂ dudit métal M avec un ligand A en présence de solvants appropriés pour dissoudre ce complexe et le ligand A, le ligand A étant choisi de façon à se transformer en ligand L au cours de la réaction,
- faire refluer cette solution sous une atmosphère d'azote, à la pression atmosphérique et à une température voisine de la température d'ébullition desdits solvants.

Comme on l'a vu précédemment, le ligand A est généralement identique au ligand L ou est de même nature que celui-ci avec des substitutions différentes. L'exemple 11 illustre ce dernier cas, par exemple.

Trois exemples de réalisation plus précis sont donnés ci-après.

### Exemple 10

### Préparation d'un complexe mono (4,4' - diméthoxy - 2,2' - bipyridine) bis (4,4' - diméthyl - 2,2' - bipyridine) d'osmium.

On fait réagir 0,05 g (0,08 mmol) d'un complexe bis (4,4' - diméthyl - 2,2' - bipyridine) Cl₂ d'osmium dont la préparation est connue en soi avec 0,022 g (1,0 mmol) de 4,4' - diméthoxy - 2,2' - bipyridine dans un mélange de 10 ml de DMF (diméthylformamide), 10 ml d'eau et 10 ml de méthanol. On fait refluer la solution pendant au moins 40 heures sous une atmosphère d'azote, à la pression atmosphérique et à une température comprise entre 70 et 80°C. Le mélange réactionnel est refroidi à température ambiante, filtré et le solvant est complètement éliminé par évaporation rotative.

Le produit est dissous dans un minimum d'éthanol, puis précipité sous forme de trifluorométhansulfonate par addition d'acide trifluorométhansulfonique. Le complexe obtenu sous forme d'un précipité est filtré avec un mélange d'acétone et de diéthyléther (1:6 vol/vol), puis séché sous vide élevé.

Ce complexe présente les propriétés physico-chimiques suivantes :

### - Potentiel d'oxydo-réduction E° (potentiel normal)

Lorsque l'on dissout le complexe dans un solvant organique tel que l'acétonitrile en présence d'un sel de LiClO₄ 0,2 M et que l'on effectue une mesure potentiodynamique avec une électrode en carbone vitreux, on obtient E° = + 500 mV (électrode de référence au calomel SCE).

Dans un tampon phosphate PBS (NaCl 100mM, NaH₂PO₄ 10mM, ajusté à pH 7,4) avec une électrode en carbone vitreux, on obtient E° = + 390 mV (électrode de référence au calomel SCE).

### - Données du spectre d'absorption des ondes UV et visibles

| longueur d'onde λ max (nm) | coefficient d'extinction ε(M⁻¹ cm⁻¹) |
|---|---|
| 222 | 70248 |
| 290 | 61806 |
| 340 | 13919 |
| 380 | 13884 |
| 452 | 12113 |
| 498 | 12119 |
| 624 | 4763 |
| 684 | 4061 |

### Exemple 11

### Préparation d'un complexe mono (4,4' - dihydroxy - 2,2' - bipyridine) bis (4,4' - diméthyl - 2,2' - bipyridine) d'osmium.

Le procédé est semblable à celui décrit dans l'exemple 10, sauf que l'on fait réagir 0,05 g (0,08 mmol) de complexe bis (4,4' - diméthyl - 2,2' - bipyridine) Cl₂ d'osmium avec 0,0195 g (0,09 mmol) de 4,4' - diméthoxy - 2,2' - bipyridine dans 10 ml d'éthylène glycol et que l'on fait refluer sous une atmosphère d'azote pendant au moins 16 heures, à la pression atmosphérique et à une température comprise entre 190 et 200°C, de préférence à 195°C.

### Exemple 12

### Préparation d'un complexe mono (4,4' - bis (N,N - éthylamino) - 2,2' - bipyridine) bis (4,4' - diméthyl - 2,2' - bipyridine) d'osmium.

On fait réagir 0,05 g (0,08 mmol) d'un complexe bis (4,4' - diméthyl - 2,2' - bipyridine) Cl₂ d'osmium dont la préparation connue en soi, avec 0,026 g (0,08 mmol) de 4,4' - bis (N,N - éthylamino) - 2,2' - bipyridine dans 10 ml d'éthylène glycol. On fait refluer la solution pendant au moins 6 heures sous une atmosphère d'azote, à la pression atmosphérique et à une température comprise entre 190 et 200°C, de préférence à 195°C. Le mélange réactionnel est refroidi à température ambiante, filtré et le solvant est concentré à la moitié de son volume d'origine. On fait précipiter le produit sous forme de trifluorométhansulfonate par addition d'acide trifluorométhansulfonique dilué. Le précipité obtenu est filtré, lavé avec un mélange (1:6 vol/vol) d'acétone et de diéthyléther, puis séché sous vide élevé.

Ce qui vient d'être décrit dans les exemples 10, 11 et 12 pour l'osmium peut être généralisé au fer, au ruthénium et au vanadium. Dans les exemples 1 à 3 et 7 à 12, on isole le produit final par addition d'acide trifluorométhansulfonique. Toutefois, il est bien évident que l'on pourrait obtenir d'autres sels en choisissant des réactifs appropriés. Ainsi, on pourrait obtenir des hexafluorophosphates par addition d'hexafluorophosphate de potassium. Dans le cas de l'osmium et de l'emploi de K₂OsCl₆ comme produit de départ, on pourrait obtenir des chlorures par addition de diéthyléther.

### Exemple 13

### Préparation d'un complexe tris (4,4'-diamino-2,2'bipyridine) de ruthénium).

On dissout 0,1g (0,38 mmol) de 4,4'-diamino-2,2'-bipyridine. On laisse refluer cette solution pendant au moins 4 heures, sous une atmosphère d'azote, à la pression atmosphérique et à une température comprise entre 190 et 200°C, de préférence à 195°C. Après refroidissement à température ambiante, on ajoute 5 ml d'acétone et 20 ml de diéthyléther. L'éther est ensuite séparé et enlevé. On a à nouveau ajouté 20 ml d'éther et répété ce processus jusqu'au point où le complexe est précipité. On isole ce complexe par filtration, on le lave avec de l'éther et on le sèche sous vide élevé.

### - Potentiel d'oxydo-réduction E°(potentiel normal)

Lorsque l'on dissout le complexe dans un solvant organique tel que l'acétonitrile en présence de LiClO₄ (0,2 M) et que l'on effectue une mesure potentiodynamique avec une électrode en carbone vitreux : on obtient E° = + 450 mV (référence : électrode au calomel (SCE)). Dans un tampon phosphate PBS (NaCl 100 mM, NaH₂PO₄ 10 mM, ajusté à pH 7,4), avec une électrode en carbon vitreux : on obtient E° = + 170 mV (référence : électrode au calomel (SCE)).

### - Données du spectre d'absorption des ondes UV et visibles

| longueur d'onde λ max (nm) | coefficient d'extinction ε(M⁻¹ cm⁻¹) |
|---|---|
| 354 | 17655 |
| 394 | 11988 |
| 472 | 8064 |
| 514 | 9444 |

### Exemple 14

### Préparation d'un complexe tris (4,4'-diamino-2,2'-bipyridine) de fer.

On dissout 0,1 g (0,50 mmol) de FeCl₂.4H₂O dans 10 ml d'eau. Cette solution est ajoutée, goutte à goutte, à une solution d'éthanol à ébulition (10 ml) qui contient 0,29 g (1,55 mmol) de 4,4'-diamino-2,2'-bipyridine. Une couleur pourpre apparait. Après refroidissement à température ambiante, on fait précipiter le complexe par l'addition de diéthyl éther (20 ml). On isole ce complexe par filtration, on le lave avec un mélange 1:6 vol/vol d'acétone et diéthyléther, et on le sèche sous vide élevé.

### - Potentiel d'oxydo-réduction E°(potentiel normal)

Lorsque l'on dissout le complexe dans un solvant organique tel que l'acetonitrile en présence de LiClO₄ (0,2 M) et que l'on effectue une mesure potentiodynamique avec une électrode en carbone vitreux : on obtient E° = + 225 mV (référence : électrode au calomel (SCE)). Dans un tampon phosphate PBS (NaCl 50 mM, NaH₂PO₄ 5 mM, ajusté à pH 7,4), avec une électrode en carbone vitreux : on obtient E° = + 70 mV) référence : électrode au calomel (SCE)).

### - Données du spectre d'absorption des ondes UV et visibles

| longueur d'onde λ max (nm) | coefficient d'extinction ε(M⁻¹ cm⁻¹) |
|---|---|
| 252 | 110800 |
| 366 | 24800 |
| 494 | 7780 |
| 580 | 10600 |

## Revendications

1. Application d'un complexe mono, bis ou tris (2,2'- bipyridine substituée) d'un métal M, comme médiateur pour capteur ampérométrique, caractérisée en ce que ledit complexe correspond à la formule générale (I) suivante 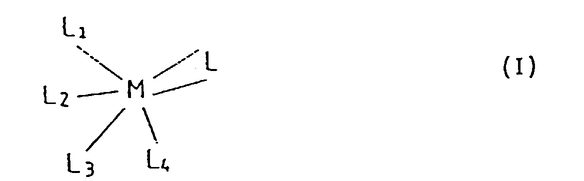 dans laquelle M est choisi parmi le fer, le ruthénium, l'osmium ou le vanadium, L représente une 2,2'-bipyridine substituée par au moins un groupe donneur d'électrons choisi parmi un groupe OH, un groupe alkoxy, un groupe aryloxy ou un groupe amine primaire, secondaire ou tertiaire, et L₁, L₂, L₃, L₄ sont des ligands formant un complexe de coordination avec le métal M.

2. Application d'un complexe selon la revendication 1, caractérisée en ce que L représente la 4,4'- dihydroxy - 2,2' - bipyridine, le complexe présentant alors la formule générale (II)

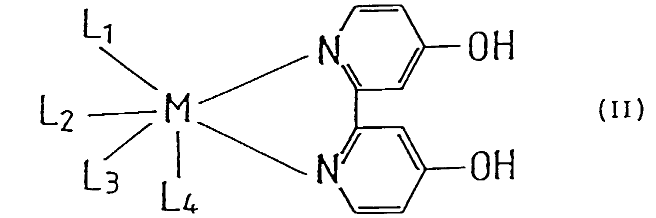

3. Application d'un complexe selon la revendication 1, caractérisée en ce que L représente la 4,4'- diméthoxy - 2,2'- bipyridine, le complexe présentant alors la formule générale (III)

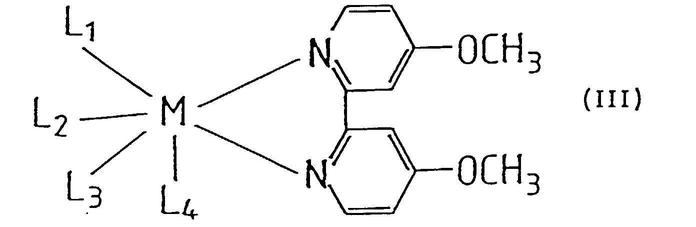

4. Application d'un complexe selon la revendication 1, caractérisée en ce que L représente la 4,4' - bis (N,N - éthylamino) - 2,2'- bipyridine, le complexe présentant alors la formule générale (IV)

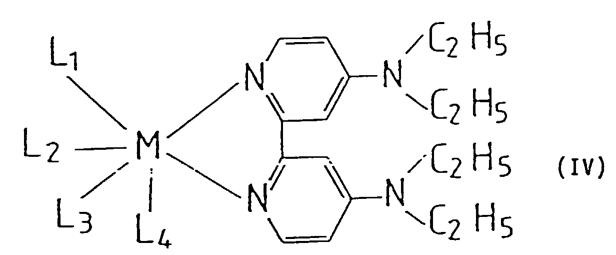

5. Application d'un complexe selon l'une quelconque des revendications 1 à 4, caractérisée en ce que L₁ et L₂ forment ensemble un ligand bidenté choisi parmi l'éthylène diamine, le 1,2 - bis (2 - pyridyl) éthane, l'acide oxalique, l'acétylacétone, la glycine ou les bipyridines et les phénantrolines bisubstituées par un groupe R₁ et un groupe R₂, R₁ et R₂ étant identiques ou différents et représentant chacun l'hydrogène, NO2, Cl, un groupe alkyle, un groupe aryle, un groupe OH, un groupe alkoxy, un groupe aryloxy ou un groupe amine primaire, secondaire ou tertiaire et en ce que L₃ et L₄ sont identiques ou différents et représentent chacun un ligand monodenté choisi parmi CN⁻, Cl⁻, Bᵣ ⁻, I⁻, SCN⁻, H₂O, NH₃, la triphénylphosphine, les trialkylphosphines, les amines primaires, secondaires ou tertiaires, la pyridine ou les pyridines substituées par Cl, NH₂, NO₂ ou un groupe alkyle.

6. Application d'un complexe selon la revendication 5, caractérisée en ce que L₁ et L₂ forment ensemble un ligand identique à L et en ce que L₃ et L₄ sont identiques à Cl⁻.

7. Application d'un complexe selon l'une quelconque des revendications 1 à 4, caractérisée en ce que L₁ et L₂ d'une part et L₃ et L₄ d'autre part forment respectivement un ligand bidenté, ces deux ligands bidentés pouvant être identiques ou différents, et étant choisis parmi l'éthylène diamine, le 1,2 - bis (2 - pyridyl) éthane, l'acide oxalique, l'acétylacétone, la glycine ou les bipyridines et les phénanthrolines, bisubstituées par un groupe R₁ et un groupe R₂, R₁ et R₂ étant identiques ou différentes et représentant chacun l'hydrogène, NO₂, Cl, un groupe aryle, un groupe OH, un groupe alkoxy, un groupe aryloxy ou un groupe amine primaire, secondaire ou tertiaire.

8. Application d'un complexe selon la revendication 7, caractérisée en ce qu'il est choisi parmi les complexes tris (4,4' - dihydroxy - 2,2' - bypiridine) du métal M, les complexes tris (4,4' - diméthoxy - 2,2' - bipyridine) du métal M ou les complexes tris (4,4' - bis (N,N - éthylamino) - 2,2' - bipyridine) du métal M, M étant choisi parmi le fer, le ruthénium, l'osmium ou le vanadium.

9. Application d'un complexe selon les revendications 2 et 7, caractérisée en ce que L₁ et L₂ forment ensemble la 4, 4' - dihydroxy - 2,2' - bipyridine et en ce que L₃ et L₄ forment ensemble un ligand bidenté choisi parmi l'éthylène diamine, le 1,2 - bis (2 - pyridyl) éthane, l'acide oxalique, l'acétylacétone, la glycine ou les bipyridines et les phénanthrolines bisubstituées par un groupe R₁ et un groupe R₂, R₁ et R₂ étant identiques ou différentes et représentant chacun l'hydrogène, NO₂, Cl, un groupe alkyle, un groupe aryle, un groupe OH, un groupe alkoxy, un groupe aryloxy ou un groupe amine primaire, secondaire ou tertiaire.

10. Application d'un complexe selon la revendication 9, caractérisée en ce qu'il est choisi parmi les complexes bis (4,4' - dihydroxy - 2,2' - bipyridine) mono (2,2' - bipyridine) du métal M, les complexes bis (4,4' - dihydroxy - 2,2' - bipyridine) mono (4,4' - diméthoxy - 2,2' - bipyridine) du métal M, les complexes bis (4,4' - dihydroxy - 2,2' - bipyridine) mono (4,4' - diméthyl - 2,2' - bipyridine) du métal M, les complexes bis (4,4' - dihydroxy - 2,2' - bipyridine) mono (4 - alkyl - 2,2' - bipyridine) du métal M, les complexes bis (4,4' - dihydroxy - 2,2' - bipyridine) mono (4,4' - dialkyl - 2,2' - bipyridine) du métal M, les complexes bis (4,4' - dihydroxy - 2,2' - bipyridine) mono (4,7 - dihydroxy - 1,10 - phénanthroline) du métal M ou les complexes bis (4,4' - dihydroxy - 2,2' - bipyridine) mono (4,4' - bis (N,N - éthylamino) - 2,2' - bipyridine) du métal M.

11. Application d'un complexe selon les revendications 3 et 7, caractérisée en ce que L₁ et L₂ forment ensemble la 4,4' - diméthoxy - 2,2' - bipyridine et en ce que L₃ et L₄ forment ensemble un ligand bidenté choisi parmi l'éthylène diamine, le 1,2 - bis (2 - pyridyl) éthane, l'acide oxalique, l'acétylacétone, la glycine ou les bipyridines et les phénanthrolines bisubstituées par un groupe R₁ et un groupe R₂, R₁ et R₂ étant identiques ou différentes et représentant chacun l'hydrogène, NO₂, Cl, un groupe alkyle, un groupe aryle, un groupe OH, un groupe alkoxy, un groupe aryloxy ou un groupe amine primaire, secondaire ou tertiaire.

12. Application d'un complexe selon la revendication 11, caractérisée en ce qu'il est choisi parmi les complexes bis (4,4' - diméthoxy - 2,2' - bipyridine) mono (2,2' - bipyridine) du métal M, les complexes bis (4,4' - diméthoxy - 2,2' - bipyridine) mono (4,4' - diméthyl - 2,2' - bipyridine) du métal M, les complexes bis (4,4' - diméthoxy - 2,2' - bipyridine) mono (4 - alkyl - 2,2' - bipyridine) du métal M, les complexes bis (4,4' - diméthoxy - 2,2' - bipyridine) mono (4,4' - dialkyl - 2,2' bipyridine) du métal M, les complexes bis (4,4' - diméthoxy - 2,2' - bipyridine) mono (4,7 - dihydroxy - 1, 10 - phénanthroline) du métal M, les complexes bis (4,4' - diméthoxy - 2,2' - bipyridine) mono (4,4' - dihydroxy - 2,2' - bipyridine) du métal M ou les complexes bis (4,4' - diméthoxy - 2,2' - bipyridine) mono (4,4' - bis (N,N - éthylamino) - 2,2' - bipyridine) du métal M.

13. Application d'un complexe selon les revendications 4 et 7, caractérisée en ce que L₁ et L₂ forment ensemble la 4,4' - bis (N,N - éthylamino - 2,2' - bipyridine et en ce que L₃ et L₄ forment ensemble un ligand bidenté choisi parmi l'éthylène diamine, le 1,2 - bis (2 - pyridyl) éthane, l'acide oxalique, l'acétylacétone, la glycine ou les bipyridines et les phénanthrolines bisubstituées par un groupe R₁ et un groupe R₂, R₁ et R₂ étant identiques ou différentes et représentant chacun l'hydrogène, NO₂, Cl, un groupe alkyle, un groupe aryle, un groupe OH, un groupe alkoxy, un groupe aryloxy ou un groupe amine primaire, secondaire ou tertiaire.

14. Application d'un complexe selon la revendication 13, caractérisée en ce qu'il est choisi parmi les complexes bis (4,4' - bis (N,N - éthylamino) - 2,2' bipyridine) mono (2,2' - bipyridine) du métal M, les complexes bis (4,4' bis (N,N - éthylamino) - 2,2' - bipyridine) mono (4,4' - diméthoxy - 2,2' - bipyridine) du métal M, les complexes bis (4,4' - bis (N,N - éthylamino) - 2,2' - bipyridine) mono (4,4' - diméthyl - 2,2' - bipyridine) du métal M, les complexes bis (4,4' - bis (N,N - éthylamino) - 2,2' - bipyridine) mono (4 - alkyl - 2,2' - bipyridine) du métal M, les complexes bis (4,4' - bis (N,N - éthylamino) - 2,2' - bipyridine) mono (4,4' - dialkyl - 2,2' - bipyridine) du métal M, les complexes bis (4,4' - bis (N,N - éthylamino) - 2,2' - bipyridine) mono (4,4' - bis (N,N - dihydroxy - 2,2' - bipyridine) du métal M ou les complexes bis (4,4' - bis (N,N - éthylamino) - 2,2' - bipyridine) mono (4,7 - dihydroxy - 1,10 - phénanthroline) du métal M.

15. Application d'un complexe selon les revendications 2 et 7, caractérisée en ce que le L₁ et L₂ d'une part et L₃ et L₄ d'autre part forment respectivement un ligand bidenté, ces deux ligands étant identiques et étant choisis parmi l'éthylène diamine, le 1,2 - bis (2, piridyl) éthane, l'acide oxalique, l'acétylacétone, la glycine ou les bipyridines et les phénanthrolines bisubstituées par un groupe R₁ et un groupe R₂, R₁ et R₂ étant identiques ou différentes et représentant chacun l'hydrogène, NO₂, Cl, un groupe alkyle, un groupe aryle, un groupe OH, un groupe alkoxy, un groupe aryloxy ou un groupe amine primaire, secondaire ou tertiaire.

16. Application d'un complexe selon la revendication 15, caractérisée en ce qu'il est choisi parmi les complexes mono (4,4' - dihydroxy - 2,2' - bipyridine) bis (4,4' - diméthyl - 2,2'- bipyridine) du métal M, les complexes mono (4,4' - dihydroxy - 2,2' - bipyridine) bis (2,2' - bipyridine) du métal M, les complexes mono (4,4' - dihydroxy - 2,2' - bipyridine) bis (4 - alkyl - 2,2' - bipyridine) du métal M ou les complexes mono (4,4' dihydroxy - 2,2' - bipyridine) bis (4,7 - dihydroxy - 1,10 - phénanthroline) du métal M.

17. Application d'un complexe selon les revendications 3 et 7, caractérisée en ce que le L₁ et L₂ d'une part et L₃ et L₄ d'autre part forment respectivement un ligand bidenté, ces deux ligands étant choisis parmi l'éthylène diamine, le 1,2 - bis (2 - pyridyl) éthane, l'acide oxalique, l'acétylacétone, la glycine ou les bipyridines et les phénanthrolines bisubstituées par un groupe R₁ et un groupe R₂, R₁ et R₂ étant identiques ou différentes et représentant chacun l'hydrogène, NO₂, Cl, un groupe alkyle, un groupe aryle, un groupe OH, un groupe alkoxy, un groupe aryloxy ou un groupe amine primaire, secondaire ou tertiaire.

18. Application d'un complexe selon la revendication 17, caractérisée en ce qu'il est choisi parmi les complexes mono (4,4' - diméthoxy - 2,2' - bipyridine) bis (4,4' - diméthyl - 2,2' - bipyridine) du métal M, les complexes mono (4,4' - diméthoxy - 2,2' - bipyridine) bis (2,2' - bipyridine) du métal M, les complexes mono (4,4' - diméthoxy - 2,2' - bipyridine) bis (4 - alkyl - 2,2' - bipyridine) du métal M, les complexes mono (4, 4' - diméthoxy - 2,2' - bipyridine) bis (4,7 - dihydroxy - 1,10 - phénanthroline) du métal M ou les complexes mono (4,4' - diméthoxy - 2,2' - bipyridine) mono (4,4' - dihydroxy - 2,2' - bipyridine) mono (4,4' - diméthyl - 2, 2 ' - bipyridine) du métal M.

19. Application d'un complexe selon les revendications 4 et 7, caractérisée en ce que le L₁ et L₂ d'une part et L₃ et L₄ d'autre part forment respectivement un ligand bidenté, ces deux ligands étant choisis parmi l'éthylène diamine, le 1,2 - bis (2 - pyridyl) éthane, l'acide oxalique, l'acétylacétone, la glycine ou les bipyridines et les phénanthrolines bisubstituées par un groupe R₁ et un groupe R₂, R₁ et R₂ étant identiques ou différentes et représentant chacun l'hydrogène, NO₂, Cl, un groupe alkyle, un groupe aryle, un groupe OH, un groupe alkoxy, un groupe aryloxy ou un groupe amine primaire, secondaire ou tertiaire.

20. Application d'un complexe selon la revendication 19, caractérisée en ce qu'il est choisi parmi les complexes mono (4,4' bis (N,N - éthylamino) - 2,2' - bipyridine) bis (4,4' - diméthyl - 2,2' - bipyridine) du métal M, les complexes mono (4,4' bis (N,N - éthylamino) - 2,2' - bipyridine) bis (2,2' - bipyridine) du métal M, les complexes mono (4,4' bis (N,N - éthylamino) - 2,2 ' - bipyridine) bis (4 - alkyl - 2,2' - bipyridine) du métal M, les complexes mono (4,4' bis (N,N - éthylamino) - 2,2' - bipyridine) bis (4,7 - dihydroxy - 1,10 - phénanthroline) du métal M.

21. Application d'un complexe selon la revendication 20, caractérisée en ce que le métal M est l'osmium.

22. Application d'un complexe selon la revendication 20, caractérisée en ce que le métal M est le ruthénium.

23. Application d'un complexe selon la revendication 20, caractérisée en ce que le métal M est le fer.

24. Complexe mono, bis ou tris (4,4' disubstitué - 2,2' bipyridine) d'osmium, caractérisé par la formule générale (I) suivante 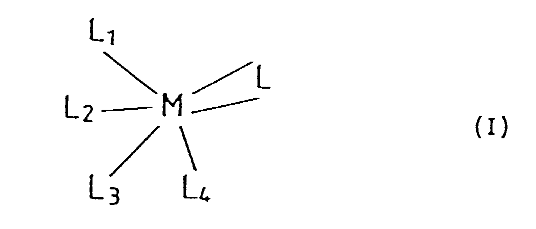 dans laquelle M représente l'osmium, L représente une 2,2' - bipyridine 4,4' disubstituée par deux groupes donneurs d'électrons choisis parmi les groupe OH, méthoxy ou diéthylamino, L₁ et L₂ d'une part et L₃ et L₄ d'autre part représentent respectivement un 2,2' - bipyridine 4,4' disubstituée par des groupes identiques à ceux de L, lesdits bipyridines disubstituées L, L₁-L₂, et L₃-L₄ pouvant être identiques entre elles, toutes différentes ou une seule différente des deux autres.

25. Procédé de préparation d'un complexe selon la revendication 24, caractérisé en ce qu'on fait agir un sel soluble au métal M successivement avec des quantités stoechiométriquement nécessaires de ligands L, L₁-L₂, et L₃-L₄, en chauffant le mélange au sein d'un solvant organique.

## Patentansprüche

1. Verwendung eines Mono-, Bis- oder Tris-Komplexes von substituiertem 2,2'-Bipyridin mit einem Metall M als Mittler für einen amperometrischen Sensor, dadurch gekennzeichnet, daß dieser Komplex der folgenden allgemeinen Formel (I) 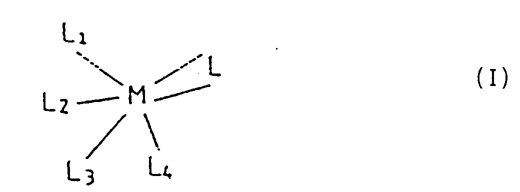 entspricht, in der M unter Eisen, Ruthenium, Osmium oder Vanadium ausgewählt ist, L einen 2,2'-Bipyridinliganden darstellt, der mit wenigstens einer Elektronendonorgruppe substituiert ist, die unter OH-, Alkoxy-, Aryloxy- oder primären, sekundären oder tertiären Amingruppen ausgewählt ist, und L₁, L₂, L₃ und L₄ Liganden sind, die mit dem Metall M einen Koordinationskomplex bilden.

2. Verwendung eines Komplexes nach Anspruch 1, dadurch gekennzeichnet, daß L für 4,4'-Dihydroxy-2,2'-bipyridin steht und der Komplex daher der allgemeinen Formel (II) entspricht

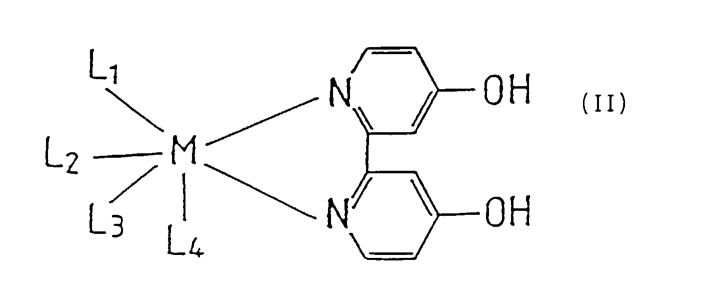

3. Verwendung eines Komplexes nach Anspruch 1, dadurch gekennzeichnet, daß L für 4,4'-Dimethoxy-2,2'-bipyridin steht und der Komplex daher der allgemeinen Formel (III) entspricht

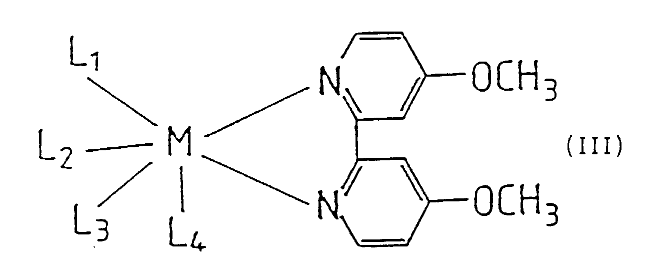

4. Verwendung eines Komplexes nach Anspruch 1, dadurch gekennzeichnet, daß L für 4,4'-Bis-(N,N-ethylamino)-2,2'-bipyridin steht und der Komplex daher der allgemeinen Formel (IV) entspricht

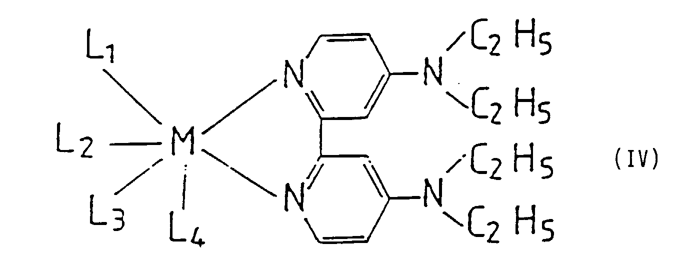

5. Verwendung eines Komplexes nach einem der Ansprüche 1 bis 4, dadurch gekennzeichnet, daß L₁ und L₂ gemeinsam einen zweizähnigen Liganden bilden, der unter Ethylendiamin, 1,2-Bis-(2-pyridyl)ethan, Oxalsäure, Acetylaceton, Glycin oder bisubstituierten Bipyridinen oder Phenanthrolinen ausgewählt ist, die mit einer Gruppe R₁ und einer Gruppe R₂ substituiert sind, wobei R₁ und R₂ gleich oder verschieden sind und jeweils Wasserstoff, NO₂, Cl, eine Alkylgruppe, eine Arylgruppe, eine OH-Gruppe, eine Alkoxygruppe, eine Aryloxygruppe oder eine primäre, sekundäre oder tertiäre Amingruppe darstellen, und daß L₃ und L₄ gleich oder verschieden sind und jeweils einen einzähnigen Liganden darstellen, der unter CN⁻, Cl⁻, Br⁻, I⁻, SCN⁻, H₂O, NH₃, Triphenylphosphin, Trialkylphosphinen, primären, sekundären oder tertiären Aminen, Pyridin und mit Cl, NH₂, NO₂ oder einer Alkylgruppe substituierten Pyridinen ausgewählt ist.

6. Verwendung eines Komplexes nach Anspruch 5, dadurch gekennzeichnet, daß L₁ und L₂ gemeinsam einen Liganden bilden, der mit L identisch ist, und daß L₃ und L₄ gleich Cl⁻ sind.

7. Verwendung eines Komplexes nach einem der Ansprüche 1 bis 4, dadurch gekennzeichnet, daß einerseits L₁ und L₂ und andererseits L₃ und L₄ jeweils einen zweizähnigen Liganden bilden, wobei diese beiden zweizähnigen Liganden gleich oder verschieden sein können und unter Ethylendiamin, 1,2-Bis-(2-pyridyl)ethan, Oxalsäure, Acetylaceton, Glycin oder bisubstituierten Bipyridinen und Phenanthrolinen ausgewählt sind, die mit einer Gruppe R₁ und einer Gruppe R₂ bisubstituiert sind, wobei R₁ und R₂ gleich oder verschieden sind und jeweils Wasserstoff, NO₂, Cl, eine Arylgruppe, eine OH-Gruppe, eine Alkoxygruppe, eine Aryloxygruppe oder eine primäre, sekundäre oder tertiäre Amingruppe darstellen.

8. Verwendung eines Komplexes nach Anspruch 7, dadurch gekennzeichnet, daß dieser Komplex ausgewählt ist unter den Tris-Komplexen von (4,4'-Dihydroxy-2,2'-bipyridin) mit einem Metall M, Tris-Komplexen von (4,4'-Dimethoxy-2,2'-bipyridin) mit einem Metall M oder Tris-Komplexen von (4,4'-Bis-(N,N-ethylamino)-2,2'-bipyridin) mit einem Metall M, wobei M unter Eisen, Ruthenium, Osmium oder Vanadium ausgewählt ist.

9. Verwendung eines Komplexes nach den Ansprüchen 2 und 7, dadurch gekennzeichnet, daß L₁ und L₂ gemeinsam das 4,4'-Dihydroxy-2,2'-bipyridin bilden, und daß L₃ und L₄ gemeinsam einen zweizähnigen Liganden bilden, der unter Ethylendiamin, 1,2-Bis-(2-pyridyl)ethan, Oxalsäure, Acetylaceton, Glycin oder bisubstituierten Bipyridinen und Phenanthrolinen ausgewählt ist, die mit einer Gruppe R₁ und einer Gruppe R₂ bisubstituiert sind, wobei R₁ und R₂ gleich oder verschieden sind und jeweils Wasserstoff, NO₂, Cl, eine Alkylgruppe, eine Arylgruppe, eine OH-Gruppe, eine Alkoxygruppe, eine Aryloxygruppe oder eine primäre, sekundäre oder tertiäre Amingruppe darstellen.

10. Verwendung eines Komplexes nach Anspruch 9, dadurch gekennzeichnet, daß dieser Komplex unter Komplexen des Typs Bis-(4,4'-dihydroxy-2,2'-bipyridin)-mono-(2,2'-bipyridin) des Metalls M, Komplexen des Typs Bis-(4,4'-dihydroxy-2,2'-bipyridin)-mono-(4,4'-dimethoxy-2,2'-bipyridin) des Metalls M, Komplexen des Typs Bis-(4,4'-dihydroxy-2,2'-bipyridin)-mono-(4,4'-dimethyl-2,2'-bipyridin) des Metalls M, Komplexen des Typs Bis-(4,4'-dihydroxy-2,2'-bipyridin)-mono-(4-alkyl-2,2'-bipyridin) des Metalls M, Komplexen des Typs Bis-(4,4'-dihydroxy-2,2'-bipyridin)-mono-(4,4'-dialkyl-2,2'-bipyridin) des Metalls M, Komplexen des Typs Bis-(4,4'-dihydroxy-2,2'-bipyridin)-mono-(4,7-dihydroxy-1,10-phenanthrolin) des Metalls M oder Komplexen des Typs Bis-(4,4'-dihydroxy-2,2'-bipyridin)-mono-(4,4'-bis-(N,N-ethylamino)-2,2' -bipyridin) des Metalls M ausgewählt ist.

11. Verwendung eines Komplexes nach den Ansprüchen 3 und 7, dadurch gekennzeichnet, daß L₁ und L₂ gemeinsam das 4,4'-Dimethoxy-2,2'-bipyridin bilden, und daß L₃ und L₄ gemeinsam einen zweizähnigen Liganden bilden, der unter Ethylendiamin, 1,2-Bis-(2-pyridyl)ethan, Oxalsäure, Acetylaceton, Glycin oder bisubstituierten Bipyridinen und Phenanthrolinen ausgewählt ist, die mit einer Gruppe R₁ und einer Gruppe R₂ bisubstituiert sind, wobei R₁ und R₂ gleich oder verschieden sind und jeweils Wasserstoff, NO₂, Cl, eine Alkylgruppe, eine Arylgruppe, eine OH-Gruppe, eine Alkoxygruppe, eine Aryloxygruppe oder eine primäre, sekundäre oder tertiäre Amingruppe darstellen.

12. Verwendung eines Komplexes nach Anspruch 11, dadurch gekennzeichnet, daß dieser Komplex unter Komplexen des Typs Bis-(4,4'-dimethoxy-2,2'-bipyridin)-mono-(2,2'-bipyridin) des Metalls M, Komplexen des Typs Bis-(4,4'-dimethoxy-2,2'-bipyridin)-mono-(4,4'-dimethyl-2,2'-bipyridin) des Metalls M, Komplexen des Typs Bis-(4,4'-dimethoxy-2,2'-bipyridin)-mono-(4-alkyl-2,2'-bipyridin) des Metalls M, Komplexen des Typs Bis-(4,4'-dimethoxy-2,2'-bipyridin)-mono-(4,4'-dialkyl-2,2'-bipyridin) des Metalls M, Komplexen des Typs Bis-(4,4'-dimethoxy-2,2'-bipyridin)-mono-(4,7-dihydroxy-1,10-phenanthrolin) des Metalls M, Komplexen des Typs Bis-(4,4'-dimethoxy-2,2'-bipyridin)-mono-(4,4'-dihydroxy-2,2'-bipyridin) des Metalls M oder Komplexen des Typs Bis-(4,4'-dimethoxy-2,2'-bipyridin)-mono-(4,4'-bis-(N,N-ethylamino)-2,2'-bipyridin) des Metalls M ausgewählt ist.

13. Verwendung eines Komplexes nach den Ansprüchen 4 und 7, dadurch gekennzeichnet, daß L₁ und L₂ gemeinsam das 4,4'-Bis-(N,N-ethylamino)-2,2'-bipyridin bilden, und daß L₃ und L₄ gemeinsam einen zweizähnigen Liganden bilden, der unter Ethylendiamin, 1,2-Bis-(2-pyridyl)ethan, Oxalsäure, Acetylaceton, Glycin oder bisubstituierten Bipyridinen und Phenanthrolinen ausgewählt ist, die mit einer Gruppe R₁ und einer Gruppe R₂ bisubstituiert sind, wobei R₁ und R₂ gleich oder verschieden sind und jeweils Wasserstoff, NO₂, Cl, eine Alkylgruppe, eine Arylgruppe, eine OH-Gruppe, eine Alkoxygruppe, eine Aryloxygruppe oder eine primäre, sekundäre oder tertiäre Amingruppe darstellen.

14. Verwendung eines Komplexes nach Anspruch 13, dadurch gekennzeichnet, daß dieser Komplex unter Komplexen des Typs Bis-(4,4'-bis-(N,N-ethylamino)-2,2'-bipyridin)-mono-(2,2'-bipyridin) des Metalls M, Komplexen des Typs Bis-(4,4'-bis-(N,N-ethylamino)-2,2'-bipyridin)-mono-(4,4'-dimethoxy-2,2'-bipyridin) des Metalls M, Komplexen des Typs Bis-(4,4'-bis-(N,N-ethylamino)-2,2'-bipyridin)-mono-(4,4'-dimethyl-2,2'-bipyridin) des Metalls M, Komplexen des Typs Bis-(4,4'-bis-(N,N-ethylamino)-2,2'-bipyridin)-mono-(4-alkyl-2,2'-bipyridin) des Metalls M, Komplexen des Typs Bis-(4,4'-bis-(N,N-ethylamino)-2,2'-bipyridin)-mono-(4,4'-dialkyl-2,2'-bipyridin) des Metalls M, Komplexen des Typs Bis-(4,4'-bis-(N,N-ethylamino)-2,2'-bipyridin)-mono-(4,4'-bis-(N,N-dihydroxy-2,2'-bipyridin) des Metalls M oder Komplexen des Typs Bis-(4,4'-bis-(N,N-ethylamino)-2,2'-bipyridin)-mono-(4,7-dihydroxy-1,10-phenanthrolin) des Metalls M ausgewählt ist.

15. Verwendung eines Komplexes nach den Ansprüchen 2 und 7, dadurch gekennzeichnet, daß einerseits L₁ und L₂ und andererseits L₃ und L₄ jeweils einen zweizähnigen Liganden bilden, wobei diese beiden Liganden gleich sind und unter Ethylendiamin, 1,2-Bis-(2-pyridyl)ethan, Oxalsäure, Acetylaceton, Glycin oder bisubstituierten Bipyridinen und Phenanthrolinen ausgewählt sind, die mit einer Gruppe R₁ und einer Gruppe R₂ bisubstituiert sind, wobei R₁ und R₂ gleich oder verschieden sind und jeweils Wasserstoff, NO₂, Cl, eine Alkylgruppe, eine Arylgruppe, eine OH-Gruppe, eine Alkoxygruppe, eine Aryloxygruppe oder eine primäre, sekundäre oder tertiäre Amingruppe darstellen.

16. Verwendung eines Komplexes nach Anspruch 15, dadurch gekennzeichnet, daß dieser Komplex unter Komplexen des Typs Mono-(4,4'-dihydroxy-2,2'-bipyridin)-bis-(4,4'-dimethyl-2,2'-bipyridin) des Metalls M, Komplexen des Typs Mono-(4,4'-dihydroxy-2,2'-bipyridin)-bis-(2,2'-bipyridin) des Metalls M, Komplexen des Typs Mono-(4,4'-dihydroxy-2,2'-bipyridin)-bis-(4-alkyl-2,2'-bipyridin) des Metalls M oder Komplexen des Typs Mono-(4,4'-dihydroxy-2,2'-bipyridin)-bis-(4,7-dihydroxy-1,10-phenanthrolin) des Metalls M ausgewählt ist.

17. Verwendung eines Komplexes nach den Ansprüchen 3 und 7, dadurch gekennzeichnet, daß einerseits L₁ und L₂ und andererseits L₃ und L₄ jeweils einen zweizähnigen Liganden bilden, wobei diese beiden Liganden unter Ethylendiamin, 1,2-Bis-(2-pyridyl)ethan, Oxalsäure, Acetylaceton, Glycin oder bisubstituierten Bipyridinen und Phenanthrolinen ausgewählt sind, die mit einer Gruppe R₁ und einer Gruppe R₂ bisubstituiert sind, wobei R₁ und R₂ gleich oder verschieden sind und jeweils Wasserstoff, NO₂, Cl, eine Alkylgruppe, eine Arylgruppe, eine OH-Gruppe, eine Alkoxygruppe, eine Aryloxygruppe oder eine primäre, sekundäre oder tertiäre Amingruppe darstellen.

18. Verwendung eines Komplexes nach Anspruch 17, dadurch gekennzeichnet, daß dieser Komplex unter Komplexen des Typs Mono-(4,4'-dimethoxy-2,2'-bipyridin)-bis-(4,4'-dimethyl-2,2'-bipyridin) des Metalls M, Komplexen des Typs Mono-(4,4'-dimethoxy-2,2'-bipyridin)-bis-(2,2'-bipyridin) des Metalls M, Komplexen des Typs Mono-(4,4'-dimethoxy-2,2'-bipyridin)-bis-(4-alkyl-2,2'-bipyridin) des Metalls M, Komplexen des Typs Mono-(4,4'-dimethoxy-2,2'-bipyridin)-bis-(4,7-dihydroxy-1,10-phenanthrol in) des Metalls M oder Komplexen des Typs Mono-(4,4'-dimethoxy-2,2'-bipyridin)-mono-(4,4'-dihydroxy-2,2'-bipyridin)-mono-(4,4'-dimethyl-2,2'-bipyridin) des Metalls M ausgewählt ist.

19. Verwendung eines Komplexes nach den Ansprüchen 4 und 7, dadurch gekennzeichnet, daß einerseits L₁ und L₂ und andererseits L₃ und L₄ jeweils einen zweizähnigen Liganden bilden, wobei diese beiden Liganden unter Ethylendiamin, 1,2-Bis-(2-pyridyl)ethan, Oxalsäure, Acetylaceton, Glycin oder bisubstituierten Bipyridinen und Phenanthrolinen ausgewählt sind, die mit einer Gruppe R₁ und einer Gruppe R₂ bisubstituiert sind, wobei R₁ und R₂ gleich oder verschieden sind und jeweils Wasserstoff, NO₂, Cl, eine Alkylgruppe, eine Arylgruppe, eine OH-Gruppe, eine Alkoxygruppe, eine Aryloxygruppe oder eine primäre, sekundäre oder tertiäre Amingruppe darstellen.

20. Verwendung eines Komplexes nach Anspruch 19, dadurch gekennzeichnet, daß dieser Komplex unter Komplexen des Typs Mono-(4,4'-bis-(N,N-ethylamino)-2,2'-bipyridin)-bis-(4,4'-dimethyl-2,2'-bipyridin) des Metalls M, Komplexen des Typs Mono-(4,4'-bis-(N,N-ethylamino)-2,2'-bipyridin)-bis-(2,2'-bipyridin) des Metalls M, Komplexen des Typs Mono-(4,4'-bis-(N,N-ethylamino)-2,2'-bipyridin)-bis-(4-alkyl-2,2'-bipyridin) des Metalls M oder Komplexen des Typs Mono-(4,4'-bis-(N,N-ethylamino)-2,2'-bipyridin)-bis-(4,7-dihydroxy-1,10-phenanthrolin) des Metalls M ausgewählt ist.

21. Verwendung eines Komplexes nach Anspruch 20, dadurch gekennzeichnet, daß das Metall M Osmium ist.

22. Verwendung eines Komplexes nach Anspruch 20, dadurch gekennzeichnet, daß das Metall M Ruthenium ist.

23. Verwendung eines Komplexes nach Anspruch 20, dadurch gekennzeichnet, daß das Metall M Eisen ist.

24. Mono-, Bis- oder Tris-Komplexe von 4,4'-disubstituiertem 2,2'-Bipyridin mit Osmium, gekennzeichnet durch die folgende allgemeine Formel (I) 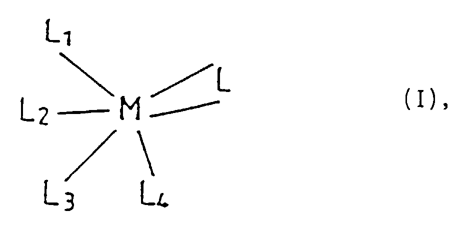 in der M für Osmium und L für ein disubstituiertes 2,2'-Bipyridin steht, das in 4,4'-Stellung mit zwei Elektronendonorgruppen versehen ist, die unter OH-Gruppen, Methoxy- oder Diethylaminogruppen ausgewählt sind, wobei L₁ und L₂ einerseits und L₃ und L₄ andererseits jeweils für ein 2,2'-Bipyridin stehen, das in 4,4'-Stellung mit den gleichen Gruppen wie L disubstituiert ist, und wobei die disubstituierten Bipyridine L, L₁-L₂ und L₃-L₄ untereinander identisch, sämtlich verschieden oder ein einzelnes verschieden von den beiden anderen sein können.

25. Verfahren zur Herstellung eines Komplexes nach Anspruch 24, dadurch gekennzeichnet, daß man ein lösliches Salz eines Metalls M nacheinander mit den notwendigen stöchiometrischen Mengen der Liganden L, L₁-L₂ und L₃-L₄ unter Erhitzen der Mischung in einem organischen Lösungsmittel zur Reaktion bringt.

## Claims

1. Application of a mono, bis or tris(substituted 2,2'-bipyridine) complex of a metal M as a mediator for an amperometric sensor, characterized in that said complex has the following general formula (I) 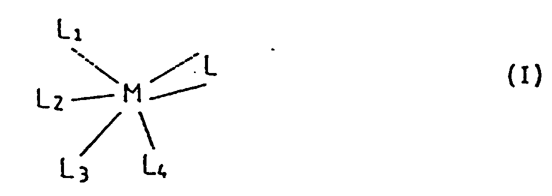 in which M represents iron, ruthenium, osmium or vanadium, L represents a 2,2'-bipyridine substituted by at least one electron donor group selected amongst a hydroxy, alkoxy, aryloxy group or a primary, secondary or tertiary amine group and L₁, L₂, L₃ and L₄ are ligands forming a coordination complex with the metal M.

2. Application of a complex according to claim 1, characterized in that L represents 4,4'-dihydroxy-2,2'-bipyridine, the complex then having the general formula II

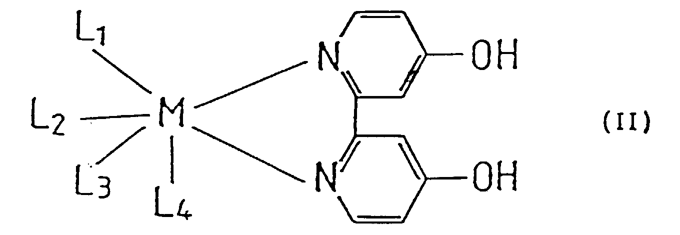

3. Application of a complex according to claim 1, characterized in that L represents 4,4'-dimethoxy-2,2'-bipyridine, the complex then having the general formula (III)

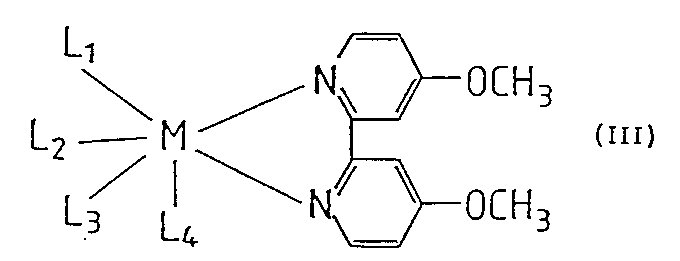

4. Application of a complex according to claim 1, characterized in that L represents 4,4'-bis(N,N-ethylamino)-2,2'-bipyridine, the complex then having the general formula (IV)

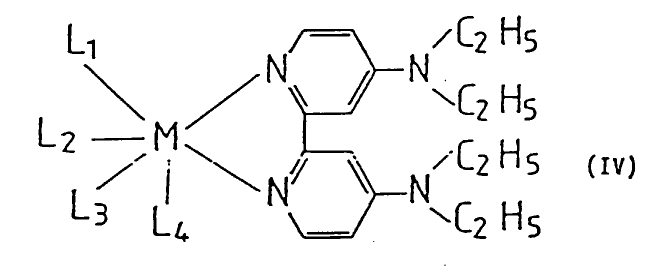

5. Application of a complex according to any one of claims 1 to 5, characterized in that L₁ and L₂ together form a bidentate ligand which is ethylenediamine, 1,2-bis (2-pyridyl)ethane, oxalic acid, acetylacetone, glycine or a bipyridine or phenanthroline disubstituted by an R₁ group and an R₂ group, R₁ and R₂ being the same or different and each representing hydrogen, NO₂, Cl, an alkyl group, an aryl group, an OH group, an alkoxy group, an aryloxy group or a primary, secondary or tertiary amine group and where L₃ and L₄ are the same or different and each represent a monodentate ligand which is CN⁻, Cl⁻, Br⁻, I⁻, SCN⁻, H₂O, NH₃, triphenylphosphine, trialkylphosphines, primary, secondary or tertiary amines, pyridine or pyridines substituted by Cl, NH₂, NO₂ or an alkyl group.

6. Application of a complex according to claim 5, characterized in that L₁ and L₂ form a ligand identical to L and in that L₃ and L₄ are identical to Cl⁻.

7. Application of a complex according to any one of claims 1 to 4, characterized in that L₁ and L₂ on the one hand and L₃ and L₄ on the other hand respectively form a bidentate ligand, these two bidentate ligands being the same or different and being ethylenediamine, 1,2-bis(2-pyridyl) ethane, oxalic acid, acetylacetone, glycine or a bipyridine or phenanthroline disubstituted by an R₁ group and an R₂ group, R₁ and R₂ being the same or different and each representing hydrogen, NO₂, Cl, an alkyl group, an aryl group, an OH group, an alkoxy group, an aryloxy group or a primary, secondary or tertiary amine group.

8. Application of a complex according to claim 7, characterized in that it is a tris(4,4'-dihydroxy-2,2'-bipyridine) complex of the metal M, a tris (4,4'-dimethoxy-2,2'-bipyridine) complex of the metal M or a tris(4,4'-bis(N,N-ethylamino)-2,2'-bipyridine) complex of the metal M, M being iron, ruthenium, osmium or vanadium.

9. Application of a complex according to claims 2 and 7, characterized in that L₁ and L₂ together form 4,4'-dihydroxy-2,2'-bipyridine and where L₃ and L₄ together form a bidentate ligand which is ethylenediamine, 1,2-bis(2-pyridyl)ethane, oxalic acid, acetylacetone, glycine or a bipyridine or phenanthroline disubstituted by an R₁ group and an R₂ group, R₁ and R₂ being the same or different and each representing hydrogen, NO₂, Cl, an alkyl group, an aryl group, an OH group, an alkoxy group, an aryloxy group or a primary, secondary or tertiary amino group.

10. Application of a complex according to claim 9, characterized in that it is a bis(4,4'-dihydroxy-2,2'-bipyridine)mono(2,2'-bipyridine) complex of the metal M, bis(4,4'-dihydroxy-2,2'-bipyridine)- mono(4,4'-dimethoxy-2,2'-bipyridine) complex of the metal M, bis(4,4'-dihydroxy-2,2'-bipyridine) mono(4,4'-dimethyl-2,2'-bipyridine) complex of the metal M, bis(4,4'-dihydroxy-2,2'-bipyridine) mono(4-alkyl-2,2'- bipyridine) complex of the metal M, the bis(4,4'-dihydroxy-2,2- bipyridine) mono(4,4'-dialkyl-2,2'-bipyridine) complexes of the metal M, the bis(4,4'-dihydroxy.2,2'-bipyridine) mono(4,7-dihydroxy-1,10-phenanthroline) complex of the metal M or the bis(4,4'-dihydroxy-1,10-phenanthroline) complex of the metal M or the bis(4,4'-dihydroxy-2,2'-bipyridine) mono(4,4'-bis(N,N-ethylamino)-2,2'-bipyridine complex of the metal M.

11. Application of a complex according to claims 3 and 7, characterized in that L₁ and L₂ together form 4,4'-dimethoxy-2,2'-bipyridine and L₃ and L₄ together form a bidentate ligand which is ethylenediamine, 1,2-bis (2-pyridyl)ethane, oxalic acid, acetylacetone, glycine or a bipyridine or phenanthroline disubstituted by an R₁ group and an R₂ group, R₁ and R₂ being the same or different and each representing hydrogen, NO₂, Cl, an alkyl group, an aryl group, an OH group, an alkoxy group, and aryloxy group or a primary, secondary or tertiary amine group.

12. Application of a complex according to claim 11, characterized in that it is a bis(4,4'-dimethoxy-2,2'-bipyridine)mono(2,2'-bipyridine) complex of the metal M; a bis(4,4'-dimethoxy-2,2'-bipyridine) mono(4,4'-dimethyl-2,2'-bipyridine) complex of the metal M; a bis(4,4'-dimethoxy-2,2'-bipyridine) mono(4-alkyl-2,2'- bipyridine) complex of the metal M; a bis(4,4'-dimethoxy-2,2'- bipyridine)mono (4,4'-dialkyl-2,2'- bipyridine) complex of the metal M; a bis(4,4'-dimethoxy-2,2'- bipyridine)mono(4,7- dihydroxy1,10-phenanthroline) complex of the metal M; a bis(4,4'-dimethoxy-2,2'-bipyridine)mono(4,4'- dihydroxy- 2,2'-bipyridine) complex of the metal M or a bis(4,4'- dimethoxy-2,2'-bipyridine)mono(4,4'-bis(N,N- ethylamino)- 2,2'-bipyridine) complex of the metal M.

13. Application of a complex according to claims 4 and 7, characterized in that L₁ and L₂ together form 4,4'-bis(N,N-ethylamino-2,2'-bipyridine) and where L₃ and L₄ together form a bidentate ligand which is ethylenediamine, 1,2-bis(2-pyridyl)ethane, oxalic acid, acetylacetone, glycine or a bipyridine or phenanthroline disubstituted by an R₁ group and an R₂ group, R₁ and R₂ being the same or different and each representing hydrogen, NO₂, Cl, an alkyl group, an aryl group, an OH group, an alkoxy group, an aryloxy group or a primary, secondary or tertiary amino group.

14. Application of a complex according to claim 13, characterized in that it is bis(4,4'-bis(N,N-ethylamino)-2,2'-bipyridine)mono(2,2'-bipyridine) complex of the metal M; a bis(4,4' bis(N,N- ethylamino)-2,2'-bipyridine)mono (4,4'-dimethoxy-2,2'- bipyridine) complex of the metal M; a bis(4,4'-bis(N,N- ethylamino-2,2'-bipyridine)mono(4,4'-dimethyl-2,2'-bipyridine) complex of the metal M; a bis(4,4'-bis(N,N- ethylamino)- 2,2'-bipyridine)mono(4-alkyl-2,2'-bipyridine) complex of the metal M; a bis(4,4'-bis(N,N-ethylamino)-2,2'-bipyridine)mono(4,4'-dialkyl-2,2'-bipyridine) complex of the metal M, bis(4,4'-bis(N,N-ethylamino)-2-2'-bipyridine)mono(4,4'- dihydroxy-2,2'-bipyridine) of the metal M or a bis(4,4'-bis(N,N-ethylamino)2,2'-bipyridine)mono(4,7-dihydroxy-1,10-phenanthroline) complex of the metal M.

15. Application of a complex according to claims 2 and 7, characterized in that L₁ and L₂ on the one hand and L₃ and L₄ on the other hand respectively form a bidentate ligand, these two ligands being the same and being ethylenediamine, 1,2 bis(2-pyridyl)ethane, oxalic acid, acetylacetone, glycine or a bipyridine or phenanthroline disubstituted by an R₁ group and an R₂ group, R₁ and R₂ being the same or different and each representing hydrogen, NO₂, Cl, an alkyl group, an aryl group, an OH group, an alkoxy group, an aryloxy group or a primary, secondary or tertiary amino group.

16. A complex according to claim 15, characterized in that it is a mono(4,4'- dihydroxy-2,2'-bipyridine)bis(4,4'-dimethyl-2,2'-bipyridine) complex of the metal M; a mono(4,4'-dihydroxy-2,2'- bipyridine)bis(2,2'-bipyridine) complex of the metal M, mono(4,4'- dihydroxy-2,2'-bipyridine)bis(4-alkyl-2,2'-bipyridine) complex of the metal M or a mono(4,4'-dihydroxy-2,2'- bipyridine)bis(4,7-dihydroxy-1,10-phenanthroline) complex of the metal M.

17. Application of a complex according to claims 3 and 7, characterized in that L₁ and L₂ on the one hand and L₃ and L₄ on the other hand respectively form a bidentate ligand, these two ligands being ethylenediamine, 1,2-bis(2-pyridyl)ethane, oxalic acid, acetylacetone, glycine or a bipyridine or phenanthroline disubstituted by an R₁ group and an R₂ group, R₁ and R₂ being the same or different and each representing hydrogen, NO₂, Cl, an alkyl group, an aryl group, an OH group, an alkoxy group, an aryloxy group or a primary, secondary or tertiary amine group.

18. A complex according to claim 17, charactrerized in that it is a mono(4,4'- dimethoxy-2,2'-bipyridine) bis(4,4'-dimethyl-2,2'-bipyridine) complex of the metal M; a mono(4,4'-dimethoxy-2,2'- bipyridine)bis(2,2'-bipyridine) complex of the metal M, mono(4,4'-dimethoxy-2,2'-bipyridine) bis(4-alkyl- 2,2'- bipyridine) complex of the metal M; a mono(4,4'-dimethoxy- 2,2'-bipyridine) bis(4,7-dihydroxy-1,10-phenanthroline) complex of the metal M or a mono(4,4'-dimethoxy-2,2'-bipyridine)mono(4,4'-dihydroxy-2,2'-bipyridine)mono(4,4'-dimethyl- 2,2'-bipyridine) complex of the metal M.

19. Application of a complex according to claims 4 and 7, characterized in that L₁ and L₂ on the one hand and L₃ and L₄ on the other hand respectively form a bidentate ligand, these two ligands being ethylenediamine, 1,2-bis(2-pyridyl)ethane, oxalic acid, acetylacetone, glycine or a bipyridine or phenanthroline disubstituted by an R₁ group and an R₂ group, R₁ and R₂ being the same or different and each representing hydrogen, NO₂, Cl, an alkyl group, an aryl group, an OH group, an alkoxy group, an aryloxy group or a primary, secondary or tertiary amine group.

20. Application of a complex according to claim 19, characterized in that it is a mono(4,4'- bis(N,N-ethylamino)-2,2'-bipyridine) bis(4,4'- dimethyl-2,2'-bipyridine) complex of the metal M; a mono(4,4'-bis(N,N-ethylamino)-2,2'-bipyridine)bis(2,2'- bipyridine) complex of the metal M; a mono(4,4'-bis(N,N-ethylamino)2,2'-bipyridine) bis(4-alkyl-2,2'-bipyridine) complex of the metal M or a mono(4,4'-bis(N,N-ethylamino)-2,2'-bipyridine)bis(4,6-dihydroxy-1,10-phenanthroline) complex of the metal M.

21. Application of a complex according to claim 20, characterized in that the metal M is osmium.

22. Application of a complex according to claim 20, characterized in that the metal M is ruthenium.

23. Application of a complex according to claim 20, characterized in that the metal M is iron.

24. A mono, bis or tris(4,4'-disubstituted - 2,2'-bipyridine) complex of osmium, characterized by the following general formula (I) 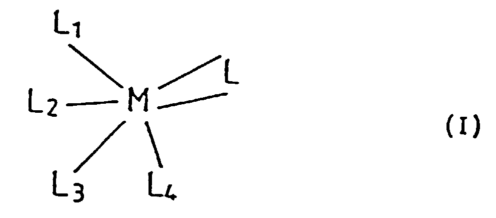 in which M represents osmium, L represents a 2,2'-bipyridine 4,4' disubstituted by at least one electron donor group selected amongst hydroxy, methoxy and diethylamino, L₁ and L₂ on the one hand and L₃ and L₄ on the other hand each being a 2,2'-bipyridine 4,4' disubstituted by the same groups as for L, said disubstituted bipyridines L, L₁-L₂ and L₃-L₄ optionnaly being all the same, all different or only one being different from the two others.

25. Process for the preparation of a complex according to claim 24, characterized in that a soluble salt of the metal M is reacted under heating in an organic solvant successively with usefull stoechiometric amounts of ligands L, L₁-L₂, and L₃-L₄.
